# EUROPEAN PATENT APPLICATION

(11) **EP 4 071 247 A1**
(43) Date of publication of application: **12.10.2022**
(21) Application number: 21846099.6
(22) Date of filing: 23.07.2021
(51) Int. Cl.: C12N 15/113, C12N 15/86, A61K 31/713, A61P 27/02

(54) **TRANS-SPLICING RIBOZYME THAT IS SPECIFIC TO RHODOPSIN TRANSCRIPTOME, AND USE THEREOF**

(30) Priority: 24.07.2020 KR 20200092265
(71) Applicant: RZNOMICS INC., Yongin-si, Gyeonggi-do 16890 (KR)
(72) Inventor: LEE, Seong-Wook, Seoul 02780 (KR); KIM, Ji Hyun, Seoul 03472 (KR); HAN, Seung Ryul, Yongin-si, Gyeonggi-do 16919 (KR)
(74) Representative: De Clercq & Partners
(86) International application number: PCT/KR2021/009555
(87) International publication number: WO 2022/019706

(57) **Abstract**

The present invention relates to a trans-splicing ribozyme targeting a rhodopsin transcript and its use for the treatment and/or prophylaxis of retinitis pigmentosa.

## Description

### Technical field

The present invention relates to a trans-splicing ribozyme targeting rhodopsin transcript and uses thereof.

### Background

Retinitis pigmentosa (RP) is a disease of the retina. It occurs due to a malfunction of photoreceptors in the retina. It is a hereditary and progressive disease in which the function of the retina, which plays a role in converting the light into electrical signals, is lost. It is characterized by progressive retinal degeneration. In the early stages of the disease, rod photoreceptor cells are damaged leading to night blindness, which suggests impairment of cell function. As the disease progresses, the visual field gradually narrows, peripheral vision may be completely lost, and ultimately, blindness may occur. Currently, no cure is available, and only treatment for retinitis pigmentosa is limited to slowing vision loss.

Retinitis pigmentosa is caused by genetic mutations in a wide variety of retinal photoreceptors. Inheritance can be autosomal dominant/recessive or X-linked. Autosomal dominant RP (ADRP, *Autosomal Dominant Retinitis Pigmentosa)* represent 15 to 25% of all RP cases. A large number of gene mutations are known to be associated with ADRP. Among them, mutations in the rhodopsin (RHO) gene account for about 30% of cases.

Rhodopsin is a G-protein coupled receptor (GPCR) present in the rod cells of the retina. As one of the light receptors, they help convert the light coming through the eye into an electrical signal and is required for enabling vision in the dark (low-light) conditions. Mutations in the RHO gene lead to functional abnormalities and deaths of rod cells, followed by rod-cone morphological abnormalities and cone cell dysfunction and death. Mutations in the RHO gene that induce RP are autosomal dominant mutation (ADRP) that cause vision loss. These mutations induce retinal degeneration and cause retinitis pigmentosa. Over 150 different missense/nonsense, insertion/deletion and splice site mutations are known as ADRP-inducing RHO mutations, and new mutations are still being reported (Non-Patent Document 1).

Among the various RHO gene mutations, the P23H mutation is the most common in ADRP patients. This mutation disrupts the folding of the RHO protein. As a result, the RHO protein is not transported to cell membrane, disrupting visual circuitry and light energy transport. Removal of the mutant allele and restoration of the normal wild-type (WT) allele expression could represent an important therapeutic strategy for treatment of ADRP.

LUXTURNA^{™} is used for the treatment of Leber congenital amaurosis (LCA) and autosomal recessive RP. LUXTURNA^{™} targets the mutation of RPE65 that has a function of maintaining photoreceptors in the eyeball. LUXTURNA^{™} has a limitation that it works only in patients with RPE65 gene mutation. Therefore, there is a need for other or new treatments for treating RP patients with mutant RHO gene.

Application of the CRISPR/cas9 system for gene editing may be possible for ADRP treatment. However, the CRISPR/cas9 system can edit a single gene mutation only. A dual delivery system for delivering a normal RHO gene is required. In addition, to correct multiple mutations, a plurality of different sgRNAs each of which target individual mutation are required. In other words, a separate system is needed to correct each individual mutation. The CRISPR/cas9 system has an additional drawback of introducing and expressing a foreign protein, such as cas9, which has a potential to induce immune response.

Gene silencing technology using siRNA/antisense RNA may be considered as another treatment option for development. However, similarly to CRISPR/cas9 system, siRNA/antisense RNA targets only a single mutation and furthermore it does not restore the RHO gene to normal. Therefore, an siRNA/antisense RNA targeting both normal and mutated RHO genes can be considered. However, this siRNA/antisense RNA system also requires a separate introduction of WT RHO gene.

As described above, an RNA-targeted method employing an antisense and a siRNA/shRNA and a gene editing method are currently under developments as treatments for RHO-ADRP. In conclusion, to overcome the drawback of these methods and to provide an ADRP treatment, a separate system for inducing WT RHO RNA expression is required.

The present inventors, as results of extensive research, developed a system capable of deleting various RHO transcripts and at the same time restoring WT RHO gene expression, by employing trans-splicing ribozyme.

[Reference Document] Non-Patent Document 1: Prog Retin Eye Res. 2018 January ; 62: 1-23.

### Detailed Description of the Invention

### Technical Objects

It is an object of the present invention to provide a trans-splicing ribozyme system that targets a rhodopsin transcript and at the same time induces wild-type (WT) RHO transcript expression.

Another object of the present invention is to provide a gene delivery system comprising the trans-splicing ribozyme system.

Another object of the present invention is to provide a genetic construct comprising the trans-splicing ribozyme system.

Another object of the present invention is to provide a recombinant expression vector comprising the genetic construct.

Another object of the present invention is to provide a recombinant virus comprising the genetic construct.

Another object of the present invention is to provide a pharmaceutical composition for preventing or treating retinitis pigmentosa comprising the trans-splicing ribozyme system, the genetic construct, the recombinant expression vector, or the recombinant virus as an active ingredient.

Another object of the present invention is to provide a method for preventing or treating retinitis pigmentosa by administering the composition to a subject.

However, the technical problem to be solved by the present invention is not limited to the above and encompasses subject matter not specifically mentioned here as will be clearly understood by those skilled in the art.

### Means of Solving Problem

In order to attain the above objects, the present invention provides trans-splicing ribozymes targeting a rhodopsin transcript. Specifically, it provides trans-splicing ribozymes capable of targeting a rhodopsin transcript and simultaneously inducing WT RHO transcript expression.

In one embodiment of the present invention, the trans-splicing ribozyme may have a structure of 5'-IGS (internal guide sequence)-Ribozyme^{∗} -3'.

In another embodiment of the present invention, the trans-splicing ribozyme may further contain an exon region at its 3' end. That is, an exon is connected to the 3-end of 5'-IGS (internal guide sequence)-Ribozyme^{∗}-3', forming 5'-IGS (internal guide sequence)-Ribozyme^{∗}-exon-3'. The exon may comprise WT rhodopsin gene transcript.

In another embodiment of the present invention, the IGS region is an oligonucleotide of 5 to 10 nucleotides (nt) in length capable of specifically binding to and forming G/U wobble base pairs with a rhodopsin transcript (RNA).

Specifically, the IGS region may comprise a nucleotide sequence of 5-10 nt in length capable of complementarily binding and forming G/U wobble base pairs at the RHO transcript regions comprising nucleotides at the following positions: +30, +35, +42, +43, +52, +54, +55, +59, +75, +97, +116, +122, +123, +127, +132, +140, +154, +165, +171, +187, +191, +207, +215, +222, +230, +232, +244, +256, +262, +273, +298, +308, +381, +403, +661 or +688, and preferably +59.

In another embodiment of the present invention, the rhodopsin transcript may contain one or more mutations.

The rhodopsin mutation(s) may a mutation(s) at any one or more bases at positions 1 to 1142 of the wild-type rhodopsin transcript of SEQ ID NO: 1. Specifically, the rhodopsin mutation(s) may include one or more known mutation(s) selected from the group consisting of the following or a mutation to be discovered in the future:
L328P, T342M, Q344R/P/ter, V345L/M, A346P, P347A/R/Q/L/S/T, ter349/Q/E, N15S, T17M, V20G, P23A/H/L, Q28H, G51R/V, P53R, T58R/M, V87D/L, G89D, G106R/W, C110F/R/S/Y, E113K, L125R, W161R, A164E/V, C167R/W, P171Q/L/S, Y178N/D/C, E181K, G182S/V, C185R, C187G/Y, G188R/E, D190N/G/Y, H211R/P, C222R, P267R/L, S270R, K296N/E/M, R135G/L/P/W, T4K, T17M, M39R, N55K, G90V, M44T, V137M, G90D, T94I, A292E, A295V, F45L, V209M, F220C, P12R, R21C, Q28H, L40R, L46R, L47R, F52Y, F56Y, L57R, Y60ter, Q64ter, R69H, N78I, L79P, L88P, T92I, T97I, V104F, G109R, G114D/V, E122G, W126L/ter, S127F, L131P, Y136ter, C140S, T160T, M163T, A169P, P170H/R, S176F, P180A/S, Q184P, S186P/W, Y191C, T193M, M207R/K, V210F, I214N, P215L/T, M216R/L/K, R252P, T289P, S297R, A298D, K311E, N315ter, E341K, S343C, and Q312ter.

In an embodiment, the ribozyme may target, but not limited to, a rhodopsin P23H mutant transcript (rhodopsin transcript with P23H mutation). The rhodopsin P23H mutation has histidine instead of proline at position 23 with respect to the normal rhodopsin protein.

The trans-splicing ribozyme may comprise or consist of any one of the nucleotide sequences represented by SEQ ID NO: 2 to SEQ ID NO: 4.

In addition, the trans-splicing ribozyme of the present invention include sequences with sequence homology of 70% or more, 80% or more, or 90% or more to the nucleotide sequence of any one of SEQ ID NOs: 2 to 4.

In an embodiment of the present invention, an antisense (AS) sequence may be linked to the 5'-end of the ribozyme.

The antisense sequence may be a sequence complementary to the target rhodopsin transcript(s).

In another embodiment of the present invention, the length of the AS region may be 10 to 210 nt in length, and preferably, 50 to 150 nt in length.

In another embodiment of the present invention, a trans-splicing ribozyme construct comprises an internal guide sequence (IGS) region consisting of or comprising the sequence of SEQ ID NO: 6, and the AS region consisting of or comprising 150 nt in length having the sequence of SEQ ID NO: 10.

According to another embodiment of the present invention, the trans-splicing ribozyme construct may have one of the following structures: 5'-IGS-Ribozyme^{∗}-3'; or 5'-AS-IGS-Ribozyme^{∗}-3'; or 5'-IGS-Ribozyme^{∗}-exon-3'; or 5'-AS-IGS-Ribozyme^{∗}-exon-3'.

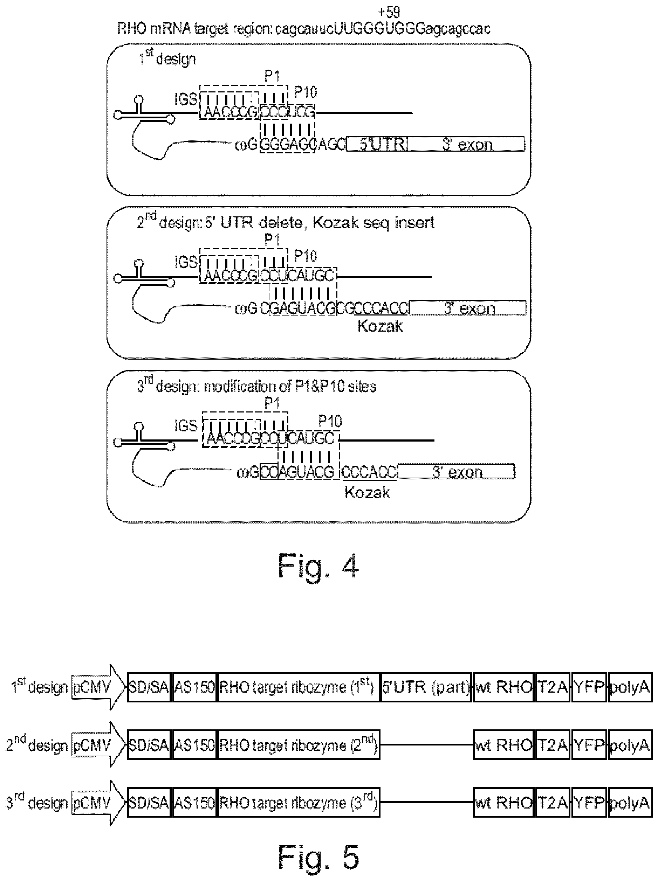

In another embodiment of the present invention, a sequence of 5 to 20 random nucleotides may be included between the IGS region and the AS region, wherein the random nucleotide sequence may be a recognition sequence for a restriction enzyme, and the random nucleotide sequence may be preferably 8 nt (nucleotide) in length, and may comprise the P1 sequence (SEQ ID NO: 7) or P10 sequence (SEQ ID NOs: 8 or 9) depicted in Figure 4.

In another embodiment of the present invention, the Ribozyme^{∗} region may comprise or consist of the sequence of SEQ ID NO: 5.

In another embodiment, the Ribozyme^{∗} region encompasses sequences having at least 70%, at least 80%, or at least 90% homology to SEQ ID NO: 5.

In another embodiment of the present invention, the exon region may contain all or part of the normal WT RHO gene sequence or all or part of an optimized RHO gene sequence.

In the present disclosure, "ribozyme" means an RNA molecule that acts like an enzyme or a molecule comprising a protein and an RNA molecule wherein RNA molecule acts like an enzyme, also known as catalytic RNA. RNA molecules with a specific tertiary structure have catalytic or autocatalytic properties, and some ribozymes inhibit activity of other RNA molecules by cleaving them while some ribozymes can inhibit their own activity by self-cleavage. Other ribozymes catalyze ribosome aminotransferase activity. These ribozymes include hammerhead ribozymes, a VS ribozyme, and hairpin ribozymes.

The present invention encompasses a ribozyme having a trans-splicing function, more specifically, trans-splicing ribozymes targeting specific variants of rhodopsin transcript.

In the present invention, "trans-splicing ribozyme" means a ribozyme (or a ribozyme construct) comprising an internal guide sequence (IGS) and a 3' exon, wherein the IGS recognizes and binds a target RNA (present separately from the ribozyme) at a specific site, cleaves the target RNA and links the 3' exon of the ribozyme immediately after the cleavage site of the target RNA.

In the present specification, the ribozyme sequence may be represented by DNA sequence or a corresponding RNA sequence. When represented by a DNA sequence, it would be obvious to one of ordinary skill in the art that an RNA sequence corresponding to the DNA sequence is also within the scope of the present invention.

In the present specification, "rhodopsin transcript(s)," "RHO transcript(s)" or "RHO RNA" means RNA molecules produced by transcription of the WT rhodopsin gene or a mutant rhodopsin gene. In the present specification, "mutant rhodopsin transcript," "mutant RHO transcript" or "mutant RHO RNA" means RNA molecules produced by transcription of a mutant rhodopsin gene (i.e., rhodopsin gene with a mutation(s)). Mutant RHO transcripts may be referred to according to the specific mutation(s) they carry, for example, "P23H RHO." Normal rhodopsin transcript may be referred to as "WT RHO transcript," "WT RHO" or "WT RHO RNA."

Trans-splicing ribozyme targeting at least one of the mutant rhodopsin transcript sites is a ribozyme that has been genetically engineered to selectively recognize and bind to a mutant rhodopsin RNA involved in retinitis pigmentosa and trigger a trans-splicing reaction when introduced into a cell. It can be manufactured by any suitable method known in the art. For example, a complementary sequence specific for a conserved region of the target RNA may be linked at the 5' end of the ribozyme making the ribozyme to have specificity to the target RNA.

A lot of information on rhodopsin mutant genes is known in the field, as summarized, for example, the above-mentioned Non-patent document 1 and website at https://www.retina-international.org/files/sci-news/rhomut.htm.

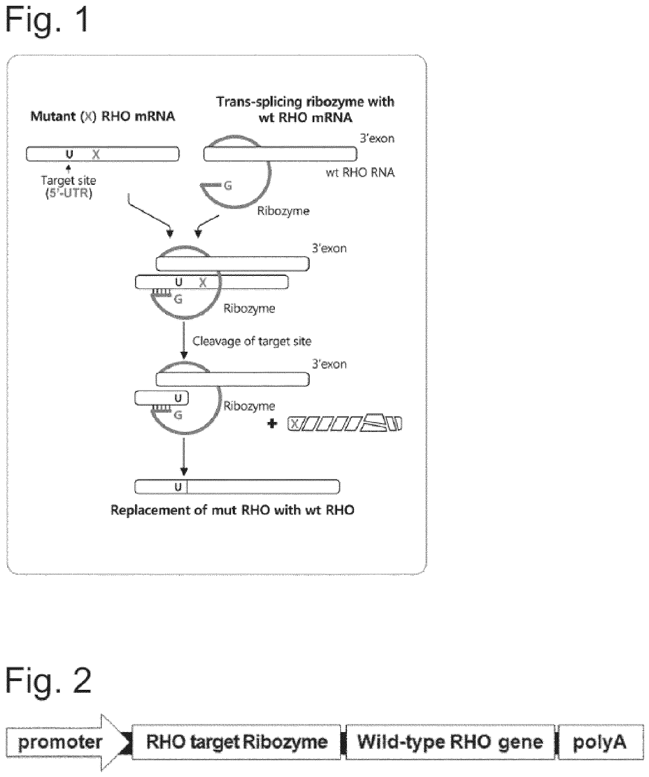

The present invention provides a system and a method to manufacture a trans-splicing ribozyme that targets rhodopsin transcripts and replace the mutant rhodopsin with the normal (WT) rhodopsin (Figure 1).

RHO mutations causing functional abnormalities occur predominantly downstream of the 5'-UTR region. The trans-splicing ribozyme according to embodiments of the present invention targets the 5'-UTR region to excise the RHO transcript region that is downstream of 5'-UTR and contains the mutation site(s) and at the same time replaces the excised RHO transcript region with a WT RHO transcript. Thus, the trans-splicing ribozyme according to the embodiments of the present invention may be used to treat or prevent RHO mutation-associated diseases such as RP regardless of mutation types.

In addition, it is possible that the trans-splicing ribozyme according to embodiments of the present invention targets the normal rhodopsin transcript. However, in this case, since the transcript is replaced with a normal rhodopsin transcript, the trans-splicing ribozymes does not affect normal rhodopsin.

In one aspect, the present invention comprises non-viral gene delivery systems for trans-splicing ribozymes whereby the trans-splicing ribozyme is loaded into a non-viral gene carrier and delivered directly into a living cell.

The non-viral gene carrier may be a liposome or lipid nanoparticles, but is not limited thereto. Any suitable delivery method known in the art may be used.

The liposome may be LIPOFECTAMINE^{®} LTX, LIPOFECTAMINE^{®} 2000, or LIPOFECTAMINE^{®} 3000, LIPOFECTIN^{®}, CELLFECTIN^{®}, LIPOFECTACE^{®}, DMRIE-C^{®}, RNAIMAX^{®}, DOTAP^{®}, SAINT-RED^{®}, AVALANCHE-OMNI^{®}, EXPLEX^{®}, POLYFECT^{®}, SUPERFECT^{®}, EFFECTENE^{®}, ATTRACTENE^{®} OR HIPERFECT^{®}. Other commercially available liposomes may also be used without restrictions.

Lipid nanoparticles include a drug and phospholipids. The phospholipids encapsulate a core formed from ionizable lipids and drug(s). They fuse to the phospholipid bilayer of the target cell and pass through the cell membrane enabling intracellular delivery of drugs and facilitating endosomal escape.

A phospholipid that can promote the fusion of lipid nanoparticles to bilayer membranes may include, but are not limited to:
dioleoylphosphatidylethanolamine (DOPE),
distearoylphosphatidylcholine (DSPC),
palmitoyloleoylphosphatidylcholine (POPC),
egg phosphatidylcholine (EPC),
dioleoylphosphatidylcholine (DOPC),
dipalmitoylphosphatidylcholine (DPPC),
dioleoylphosphatidylglycerol (DOPG),
dipalmitoylphosphatidylglycerol (DPPG),
distearoylphosphatidylethanolamine (DSPE),
Phosphatidylethanolamine (PE),
dipalmitoylphosphatidylethanolamine;
1 ,2-dioleoyl-sn-glycero-3 -phosphoethanolamine,
1-palmitoyl-2-oleoyl-sn-glycero-3-phosphoethanolamine (POPE),
1-palmitoyl-2-oleoyl-sn-glycero-3-phosphocholine (POPC),
1,2-dioleoyl-sn-glycero-3-[phospho-L-serine] (DOPS), and
1,2-dioleoyl-sn-glycero-3-[phospho-L-serine]. One or more of these phospholipids may be used

Cationic lipid transporters include a complex formed from gene molecules, expression vectors carrying a gene, or a nucleic acid molecule, which are negatively charged, and nano-sized liposomes (of cationic lipids) or lipid nanoparticles, which are positively charged. The complex is delivered into cells by phagocytosis. The complex is delivered into the cell in an endosome and is transferred into a lysosome and then exits into the cytoplasm and is expressed. Another method of delivery of nucleic acids into the cells use cationic polymers and are similar to the cationic lipid transporters, except for employing the cationic polymers in place of the cationic lipids. Representative cationic polymers include polyethylenimine, poly-L-lysine, chitosan, and the like.

The trans-splicing ribozyme according to embodiments of the present invention, which carries the desired gene such as a normal wild-type rhodopsin, can be loaded into a non-viral gene delivery system. A normal rhodopsin gene can be introduced to a target site of the RHO transcript using the non-viral gene delivery system.

In another aspect, the present invention provides a gene construct comprising the trans-splicing ribozyme.

The gene construct comprises a desired gene in the 3' exon region of the ribozyme.

The desired gene may be a normal rhodopsin gene and/or a reporter gene.

The reporter gene may be luciferase, green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescent protein (EYFP), or any other suitable reporter gene known in the art.

By inserting a reporter gene as a desired gene, the expression level of rhodopsin transcript may be observed.

The gene construct may comprise an operably linked promoter sequence.

The promoter may be promoter of prokaryotic cells or mammalian viruses such as CMV promoter (cytomegalovirus promoter), CAG promoter (CMV early enhancer element + chicken beta-actin promoter/ splicing donor (SD) + rabbit beta- globin splicer acceptor (SA)), SV40 promoter, adenovirus promoter (major late promoter), pLλ promoter, trp promoter, lac promoter, tac promoter, T7 promoter, vaccinia virus 7.5K promoter, HSV tk promoter, SV40E1 promoter, respiratory syncytial virus (RSV) promoter, and LTR promoter, or promoter of mammalian cells such as metallothionein promoter, beta-actin promoter, ubiquitin C promoter, EF1-alpha (elongation factor 1-alpha) promoter, IL-2 (interleukin-2) gene promoter, lymphotoxin gene promoter, and GM-CSF (granulocyte-macrophage colony stimulating factor) gene promoter, or retinal tissue-specific promoter such as RHO (rhodopsin) gene promoter, RK (rhodopsin kinase) gene promoter, and RedO (red opsin) gene promoter, or other suitable promoters.

As used herein, "gene construct" refers to a construct comprising an element to enhance specificity to target site(s) of target rhodopsin transcript(s). In particular, the gene construct may contain the trans-splicing ribozyme according to embodiments.

An antisense sequence linked to the trans-splicing ribozyme of the gene construct may increase specificity of ribozyme for the target rhodopsin transcript and, thus, enhance the therapeutic effect. Any sequence intended for achieving this purpose may be included without limitation.

The construct according to embodiments of the present invention targets a rhodopsin transcript and also contains an antisense sequence specific for the targeted rhodopsin transcript, and, thus, can be specifically expressed only in cells in which rhodopsin is expressed.

Figure 2 depicts a gene construct according to one embodiment of the present invention, comprising a promoter, a trans-splicing ribozyme region, and a normal rhodopsin gene region.

In another aspect of the present invention a recombinant expression vector comprising the above-mentioned gene construct is provided.

In another aspect of the present invention a recombinant virus comprising the above-mentioned gene construct is provided.

The virus may be an adenovirus, adeno-associated virus (AAV), retrovirus, lentivirus, herpes simplex virus, or vaccinia virus, or any other suitable virus.

As used herein, the term "vector" refers to a construct capable of expressing a desired protein in an appropriate host cell. An expression vector refers to a genetic construct comprising a gene insert operably linked to essential regulatory elements enabling its expression. In the present invention, the term, "operably linked" means a functional linkage between the expression regulatory sequence and the nucleic acid sequence encoding the desired transcript. For example, by operably linking the ribozyme coding sequence to a promoter, the expression of the ribozyme coding sequence is placed under the influence or control of this promoter. Operably linking the two sequences (the ribozyme coding sequence and the promoter region sequence at the 5' end of the ribozyme coding sequence) allows the ribozyme coding sequence to be transcribed by action of the promoter. If the linkage between the two sequences does not induce frame-shift mutations, and does not inhibit the expression regulatory sequence's function of controlling the ribozyme expression, the linkage is considered as "operably linked." Operable linkages within recombinant vectors are well known in the art. They can be prepared using known genetic recombination technology, e,g., using site-specific DNA enzymes for cleavage and ligation generally known in the art.

The vector of the present invention may include a promoter, an operator, a Kozak consensus sequence, a start codon, a stop codon, and other elements, such as polyadenylation signals, enhancers, membrane targeting sequence, signal sequence, or leader sequence for secretion, and may be prepared in various ways depending on the purpose. The promoter of the vector may be constitutive or inducible. In addition, the vector may contain a selectable marker for selecting host cells containing the construct, and, in the case of an expression vectors, an origin of replication. A vector may self-replicate or be incorporated into host DNA. The vector may be a plasmid vector, a cosmid vector, a viral vector, or another suitable vector.

If a vector is a viral vector, it may be derived from adenovirus, adeno-associated virus (AAV), retrovirus, lentivirus, herpes simplex virus, vaccinia virus, or other suitable viruses.

Another embodiment of the present invention is a transformed cell into which the recombinant vector is introduced.

As used herein, the term "introduction" refers to introducing a foreign gene into a cell. Constructs according to embodiments of the present invention may be introduced into a cell by transfection or transduction. Transfection may be performed by calcium phosphate-DNA coprecipitation, DEAE-dextran-mediated transfection, polybrene-mediated transfection, electroporation, microinjection, liposome fusion, lipofectamine, protoplast fusion and by various other methods known in the art. Transduction may be performed by means of infection with a virus wherein the viral particles are used to deliver genes into cells.

As used herein, the term "transformed cell" refers to a target cell into which the polynucleotide has been introduced.

Transformation can be accomplished by the methods enumerated above, or by another appropriate technique suitable for introducing genetic material into a host cell.

A transformed cell of the present invention may be obtained by introducing a recombinant vector containing a polynucleotide encoding a ribozyme targeting the rhodopsin transcript.

In one embodiment of the present invention, the recombinant virus is a recombinant adeno-associated viruses (AAV). In one embodiment of the present invention recombinant adeno-associated viruses (AAV) contains a polynucleotide sequence encoding a trans-splicing ribozyme operably linked to a promoter.

The recombinant AAV is a natural or an artificially derived serotype, isolate or a clade of AAV.

The polynucleotide sequence encoding the trans-splicing ribozyme may comprise or consist of any one of SEQ ID NOs: 2-5 and any sequences having sequence homology of 70% or more, 80% or more, or 90% or more to any one of SEQ ID NOs: 2-5.

The promoter may be a CMV promoter or a RHO promoter.

The recombinant AAV may further include regulatory sequences.

The regulatory sequence may be a splicing donor/splicing acceptor sequence (SD/SA) and/or a woodchuck hepatitis post-regulatory element (WPRE) sequence.

In the embodiments, the SD/SA can promote transcription initiation, processing of RNA polymerase II, and nucleocytoplasmic export of mRNA.

In the embodiments, WPRE sequence may be used to significantly increase the expression and function of ribozyme RNA by increasing the levels of pre-mRNA and/or promoting mRNA processing and transport from the nucleus to the cytoplasm.

The SD/SA sequence according to the present invention may correspond to the beginning and ending of the truncated intron that is spliced to be removed. The SD sequence may be a GU sequence at the 5' end of an intron and SA sequence may be an AG sequence at the 3' end of an intron.

WPRE according to the present invention refers to a sequence that increases the expression of a gene by forming a tertiary structure that promotes transcription of DNA.

In the embodiments, the SD/SA sequence and WPRE sequence may be SEQ ID NO: 11 and SEQ ID NO: 13, respectively, and may be present in the desired gene expression cassette. Other sequences promote gene expression may also be used.

The AAV may be either a naturally or artificially derived serotype or isolate or clade of AAV. Thus, the AAV genome may be a native AAV viral genome or it may be a genome or an artificially created AAV viral genome. As known to those skilled in the art, AAV viruses can be classified depend on various biological systems.

AAV viruses are generally referred to according to their serotype. The serotypes are distinguished from each other by the expression profile of their capsid surface antigens. Variant subspecies of AAV with unique reactivity may be used. In general, viruses with a specific AAV serotype are not cross-reactive with neutralizing antibodies specific for other AAV serotypes. AAV serotypes are AAV1, AAV2, AAV3, AAV4, AAV5, AAV6, AAV7, AAV8, AAV9, AAV10 and AAV11. Recombinant serotypes, such as Rec2 and Rec3, recently identified in the primate brain, are also included.

AAV serotypes of particular interest for use in the present invention are those able to infect ocular tissues, such as retinal pigment epithelium. AAV2, AAV5 and AAV8 are efficiently transduced into these tissues. A review of AAV serotypes is provided by Choi, V. W., et al. (2005), AAV hybrid serotypes: improved vectors for gene delivery [Choi et al. (Curr Gene Ther. 2005;5(3);299-310); and Wu et al. (Molecular Therapy; 14(3),316-327)].

AAV viruses are also referred to in terms of clades or clones. Viruses in the same clade or clone have a natural or artificial phylogenetic relationships. A clade or a clone generally includes a phylogenetic family of AAV viruses that can be traced to a common ancestor. AAV viruses can be found in nature or artificially engineered as a specific isolate, such as a genetic isolate of a specific AAV virus. Genetic isolate refers to a distinct population of viruses that exhibit limited mixing with other virus populations and may be recognized at a genetic level.

In general, the genome of naturally occurring or artificially derived serotypes, isolates or strains of AAV comprises one or more inverted terminal repeat sequences (ITRs). The ITR sequence provides a functional origin of replication at the cis position, and allows for fusion and cleavage of vector to/from cell genomes. In some embodiments, one or more ITR sequences flank polynucleotide sequences encoding Rep-1 or a variant thereof.

The AAV genome also generally contains packaging genes, such as rep and/or cap genes, which encode proteins necessary for packaging of AAV viral particles. The rep gene encodes at least one of Rep78, Rep68, Rep52 and Rep40 or variants thereof. The AAV genome may also contain one or more cap gene, such as VP1, VP2 and VP3 or variants thereof. Cap genes encode a capsid proteins. These proteins form the capsids of AAV viral particles. A promoter is operably linked to each packaging gene.

The AAV vectors may contain the entire genome of a natural or artificial AAV. A vector comprising the entire AAV genome may be prepared in vitro. Such vectors can in principle be administered to a patient. Preferably, the AAV genome is derivatised for administration to a patient. Such derivatisations are standard procedure in the art. In some embodiments of the present invention, a vector may be prepared by applying techniques known in the art including the use of derivatisations or modifications.

In another aspect, the present invention provides a method and a system for non-viral delivery of gene constructs discussed above.

The non-viral gene carrier may be liposomes or lipid nanoparticles, or other suitable non-viral delivery systems known in the art. Detailed description of such systems is not included here.

In its various aspects, the present invention provides a ribozyme, a non-viral gene carrier, a gene construct, a recombinant expression vector or a recombinant pharmaceutical for preventing or treating retinitis pigmentosa.

In another aspect, the present invention provides a method for preventing or treating retinitis pigmentosa comprising the step of administering a pharmaceutical composition to a subject.

As used herein, the term "prevention" refers to any action that delays the onset of retinitis pigmentosa by administration of the pharmaceutical composition according to the present invention.

As used herein, the term "treatment" refers to any action that improves or beneficially changes the course of retinitis pigmentosa by the administration of a pharmaceutical composition of the present invention.

The pharmaceutical composition according to the present invention may further contain a pharmaceutically acceptable carrier, excipient or a diluent. Examples of pharmaceutically acceptable carriers, excipients and diluents include lactose, dextrose, sucrose, sorbitol, mannitol, xylitol, erythritol, maltitol, starch, acacia gum, alginate, gelatin, calcium phosphate, calcium silicate, calcium carbonate, cellulose, methyl cellulose, polyvinylpyrrolidone, water, methylhydroxybenzoate, propyl hydroxybenzoate, talc, magnesium stearate, mineral oil and others.

The pharmaceutical composition of the present invention may be administered orally or parenterally. Parenteral administration is preferred. There are three routes for parenteral intraocular administration: intravitreal injection, suprachoroidal injection and subretinal injection.

According to one embodiment of the present invention, the pharmaceutical composition may be administered efficiently and directly to photoreceptor cells by a subretinal injection. The composition to be administered by injection may be dissolved in a sterile medium. Before administration by injection, distilled water may be added to obtain the appropriate concentration of the composition. In addition, when prepared as an injection, buffers, preservatives, analgesics, solubilizers, isotonic agents, stabilizers and other ingredients may be added. Compositions may be prepared and used in dosage forms, such as in single dose ampoules or in multiple doses.

AAV carrying the trans-splicing according to the embodiments can be used to remove a target RHO transcript and induce WT RHO in an episomal state, instead of directly inserting into human genome.

The dosage of the pharmaceutical composition of the present invention depends on the patient's condition and weight, disease stage and other drugs the patient may be taking. The dosage may vary depending on the form, administration route and timing, and may be appropriately determined by those skilled in the art. Pharmaceutical composition according to the present invention may be used alone or in combination with other therapeutic agents, or as an adjuvant, for example, after surgical treatment.

### Effects

Trans-splicing ribozyme according to the embodiments targets rhodopsin transcript and replaces it with normal (wild-type) rhodopsin RNA allowing the normal gene to be expressed. Therefore, trans-splicing ribozyme according to the embodiments may provide a curative treatment for retinitis pigmentosa. The target transcript may harbor any rhodopsin mutations. The trans-splicing ribozyme has the advantage of being applicable to all patients with various rhodopsin mutations by selecting a site of target rhodopsin transcript, which facilitates excision of rhodopsin transcript containing any mutations and replacing the excised transcript with a normal WT rhodopsin transcript. In addition, the expression of normal WT rhodopsin depends on the amount of target rhodopsin transcripts expressed in patient cells, adverse effects or toxicity caused by excessive expression of normal rhodopsin proteins can be minimized. Also the RNA restoration by trans-splicing ribozymes does not utilize endogenous cell machinery and the trans-splicing ribozyme system does not require introduction of an external protein to function within the cell. Therefore, trans-splicing ribozyme is safer.

### Brief description of the drawings

Figure 1 is a schematic diagram showing the mechanism of trans-splicing ribozyme targeting RHO transcript according to an embodiment of the present invention.
Figure 2 is a schematic diagram showing a basic configuration of a gene construct comprising a ribozyme according to an embodiment of the present invention containing a promoter, trans-splicing ribozyme sequence region, and normal (WT) rhodopsin gene sequence region.
Figure 3 is a schematic diagram showing the process and the results of selection of target sites on RHO RNA through in vitro mapping and intracellular mapping.
Figure 4 is a schematic diagram showing optimization of a gene construct comprising a trans-splicing ribozyme according to an embodiment of the present invention targeting +59 site (the base at position 59) of the Rho transcript (hereinafter referred to as "RHO targeting ribozyme").
Figure 5 is a schematic diagram of a vector including a RHO targeting ribozyme according to an embodiment of the present invention.
Figure 6 depicts the results of comparison of RHO targeting ribozyme constructs in 293A cells.
Figure 7 shows the results of an experiment confirming *in vitro* efficacy of the RHO targeting ribozyme using cell lines stably expressing the P23H mutant hRHO gene.
Figure 8 shows the results of an experiment confirming *in vitro* efficacy of RHO-targeting ribozymes against various RHO mutations.
Figure 9 is a schematic diagram of a recombinant AAV expression vector comprising a RHO targeting ribozyme according to an embodiment of the present invention.
Figure 10 shows experimental results confirming the efficacy of ribozyme in a disease mouse model using an AAV expression vector comprising a RHO targeting ribozyme according to an embodiment of the present invention (a shows the result 2 weeks after administration, and b shows the result 5 weeks after administration).
Figure 11 shows immune and inflammatory responses in serum after administration of an AAV vector containing RHO targeting ribozyme in a mouse disease model according to an embodiment of the present invention. (A is the result 2 weeks after administration, B is the result 5 weeks after administration).
Figure 12 shows experimental results confirming the efficacy of the RHO targeting ribozyme in retina and retinal pigment epithelium (RPE) in a mouse model after administration of the AAV vector according to an embodiment of the present invention.
Figure 13 shows the *in vivo* distribution of RHO target ribozyme in a disease mouse model after administration of an AAV vector comprising the ribozyme according to an embodiment of the present invention.
Figure 14 shows the result of analyzing the toxicity after administration of the AAV vector containing a RHO targeting ribozyme in a normal mouse according to an embodiment of the present invention.
Figure 15 shows the result of performing an electroretinogram test after administration of the AAV vector containing a RHO targeting ribozyme in a mouse disease model according to an embodiment of the present invention.
Figure 16 shows the results of the analysis of serum (A) and ribozyme activity (B) after administration of the AAV vector containing a RHO targeting ribozyme according to an embodiment of the present invention in a disease mouse model.
Figure 17 is a schematic diagram of various recombinant AAV vectors containing RHO targeting ribozymes according to an embodiment of the present invention.
Figure 18 shows experimental result confirming the trans-splicing activity of RHO targeting ribozyme in a disease mouse model at 3 weeks after administration of the recombinant AAV vector containing the ribozyme according to an embodiment of the present invention.
Figures 19 and 20 show electroretinograms of a disease mouse model, after administration of a recombinant AAV vector containing the RHO targeting ribozyme according to an embodiment of the present invention.
Figure 21 shows ribozyme distribution and ribozyme RNA expression in the retina after administration of a recombinant AAV vector containing the RHO targeting ribozyme according to an embodiment of the present invention.

### Modes for carrying out the embodiments

1 µg of rhodopsin RNA and 1 µg of the random library ribozyme were put in a 1.5 µl tube and mixed. Then, 100 µl of Opti-MEM was added and mixed. 2 µl of Lipofectamine 2000 and 100 µl of Opti-MEM were added. The present invention has various embodiments which may include various modifications. Hereinafter, specific embodiments illustrated in the drawings are described in detail. However, this is not intended to limit the invention to specific embodiments. All modifications, equivalents and substitutes within the spirit and scope of the present invention should be understood to be included. When a detailed description of the technology may obscure the essence of the present invention, such description may be omitted.

### Example 1: Design of Rhodopsin (RHO) RNA targeting trans-splicing ribozyme

Sequences of RHO RNA variants were analyzed to select a target RNA site. Design of RHO targeting trans-splicing ribozyme is shown in Figure 2.

### Example 2: Selection of target site of Rhodopsin (RHO) RNA by in vitro Mapping and intracellular mapping

WT RHO RNA or P23H RHO RNA and ribozyme RNA library was used to perform in vitro mapping and intracellular mapping. A reaction was carried out by mixing the random ribozyme library RNA with Rhodopsin RNA in test tubes for in vitro mapping, RT-PCR was performed and then trans-splicing was performed through sequencing of PCR DNA bands expected as products. The target rhodopsin RNA site was identified.

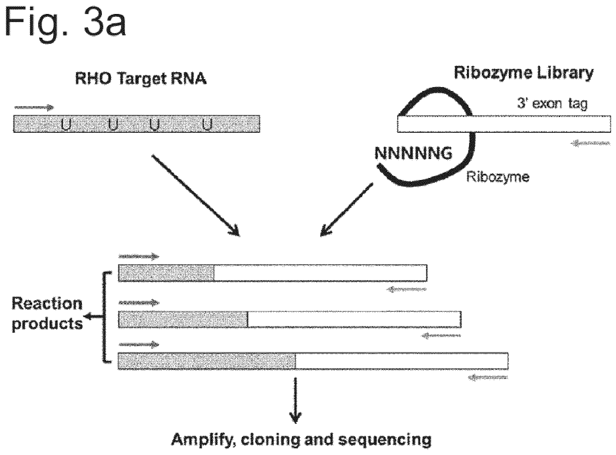

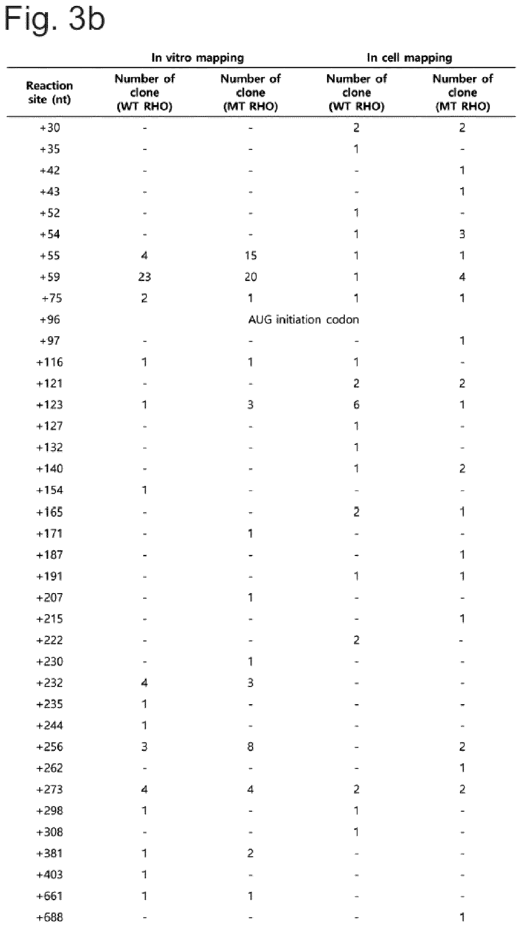

More specifically, rhodopsin RNA is synthesized by *in vitro* transcription and trans-splicing ribozyme library RNA with a random sequence (GNNNNN) at the 5' end is constructed. In vitro transcription was performed as follows: 0.1 pmol of rhodopsin RNA was added to 1X in vitro trans-splicing reaction Buffer (50 mM HEPES (pH 7.0), 150 mM NaCl, 5 mM MgCl₂) to make a total of 10 µL. 1 pmole of ribozyme library RNA was added to mix with in vitro trans-splicing reaction buffer (IX) to make a total of 9 µL, then 1 µL of 1 mM GTP was added for the final concentration of 0.1mM GTP in a separate tube. The two separate reactions were incubated at 95 °C for 1 minute and then at 37 °C for 3 minutes, respectively. The two reactions were then mixed and incubated at 37 °C for 3 hours. RNA was recovered from the reaction mix by phenol extraction/EtOH precipitation, and RT-PCR was performed. A ribozyme-specific RT primer (5'-ATGTGCTGCAAGGCGATT-3') was added, and cDNA was synthesized by reverse transcription in a total volume of 20 µL 35 cycles of PCR were carried out using 2uL of cDNA, 10 pmol of rhodopsin RNA specific 5' primer (5'- CTACTCAGCCCCAGCGGAGG-3'), and 10 pmol of ribozyme specific 3' primer (RY-TS) (5'-TGTAAAACGACGGCCAGTG-3') under the following cycling conditions: 95 °C 30 seconds, 55 °C for 30 seconds and 72 °C for 30 seconds. PCR products were separated by electrophoresis on a 2% TBE agarose gel. All identified PCR products were subjected to phenol extraction & ethanol precipitation process. After buffer change, a sequencing was performed to determine the site(s) where the trans-splicing reaction occurs in the rhodopsin RNA. Sequencing analysis of trans-splicing reaction sites revealed that most efficient targeting occurred at 59^{th} site that is the 5' UTR region of RHO RNA (Figure 3A and Figure 3B).

Intracellular co-transfection was performed by mixing random ribozyme library RNA with rhodopsin RNAs in test tube, to carry out intracellular mapping. Specifically, the 293 cells were seeded in a 35mm culture dish at a density 1×10⁵ and were maintained at 37 °C incubator with 5% CO₂. The random ribozyme library RNA and rhodopsin RNA were collected in a 1.5 ml tube and then mixed with Opti-MEM 100ul. Lipofectamin 2000 mixed with Opti-MEM 100ul and incubated at room temperature for 5 minutes. Then, the contents of the two tubes were mixed and incubated at room temperature for 20 minutes to allow for liposome complex formation. After 20 minutes, the tubes were centrifuged for 10 seconds and the liposome complex was added on the cells for co-transfection. After 4 hours, the medium was replaced with a new medium. The cells were kept in the incubator at 37° C with 5% CO₂ for 24 hours. After incubation, the cells were washed with 1X PBS, and RNA was extracted by treatment with trizol 500ul. 5 µg of the extracted RNA was treated with 1 µl of DNase I to remove gDNA. 1 µg of RNA was mixed with the ribozyme-specific RT primer (5'-ATGTGCTGCAAGGCGATT-3'), and cDNA was synthesized through reverse transcription in a volume condition of 20 uL. 2uL of synthesized cDNA was amplified with 10 pmol each of rhodopsin RNA specific 5' primer (5' CTACTCAGCCCCAGCGGAGG-3'), ribozyme specific 3' primer (RY-TS) (5'-TGTAAACGACGGCCAGTG-3') using the following cycling conditions: 35 cycles of 30 sec at 95°C, 30 sec at 55 °C, 30 sec at 72 °C. PCR products were separated on a 2% TBE agarose gel. PCR products were isolated by phenol extraction & ethanol precipitation. The nucleotide sequence analysis was performed to determine where the trans-splicing reaction occurred within RHO RNA.

### Example 3: Preparation of trans-splicing ribozyme

A gene construct containing trans-splicing ribozyme targeting the base at position 59 of the RHO transcript and WT RHO represented by SEQ ID NO: 1 was manufactured and optimized (Fig. 4 ). The target site includes a base at position 59 of the RHO RNA, and the construct includes IGS (5'-GCCCAA-3': SEQ ID NO: 6), at the 5' end, wherein the IGS is capable of targeting the above target site. To analyze the trans-splicing efficiency of the ribozyme targeting RHO RNA +59 base ("RHO target ribozyme") in cells, an improved RHO targeting ribozyme construct was prepared. It is known that Group I introns having 6-nt IGS alone are, when expressed in mammalian cells, either inactivated or exhibit non-specific activity. To prepare an improved RHO targeting ribozyme construct, a complementary oligonucleotide containing an extended P1 (SEQ ID NO: 7) and 7-nt long P10 helix (SEQ ID NO: 8 and/or SEQ ID NO: 9) was inserted to the upstream of IGS of the ribozyme.

### Example 4: Optimization of trans-splicing ribozymes

To confirm that RHO targeting ribozyme was optimized for trans-splicing, vectors were constructed with RHO targeting ribozyme of the first, second and third design prepared in Example 3 (Figure 5).

In Figure 5, AS150 is SEQ ID NO: 10; SD/SA is SEQ ID NO: 11; sequence of part of 5'UTR is SEQ ID NO: 12. Linker sequence T2A, YFP and polyA sequences are well known in the art and their specific nucleotide sequences are omitted.

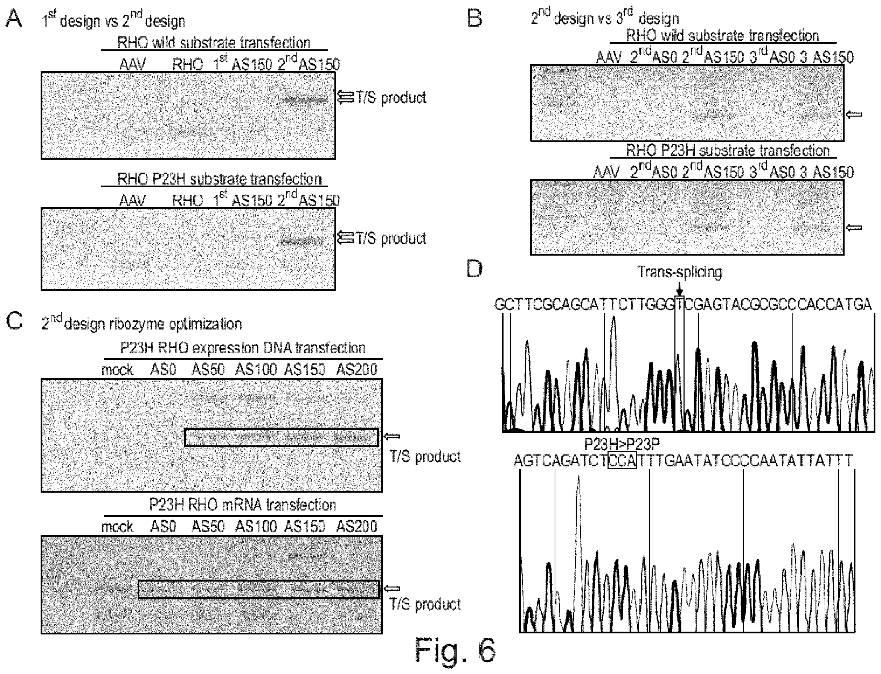

The effect of the three designs of RHO targeting ribozyme was investigated. Comparative verification was performed in vitro (in mammalian cells). The results are shown in Figure 5. Design 1 was compared to design 2 and it was confirmed that the trans-splicing efficiency of design 2 was better (Figure 6A). Comparison of designs 2 and 3 demonstrated that the trans-splicing efficiency of design 2 was better (Figure 6B). Antisense sequences of different lengths were compared to optimize the specificity and effectiveness of the ribozyme. Antisense sequence of 150 nucleotides in length (SEQ ID NO: 10) was found to be the most effective (Figure 6C). Sequencing of trans-splicing products was performed to confirm that trans-splicing was performed accurately. It was confirmed that trans-splicing occurred at the target site (Figure 6D).

### Example 5: Assay for trans-splicing activity in vitro

The *in vitro* efficacy of RHO-targeting ribozyme was confirmed using stable cells. A YFP-tagged wild-type RHO (wild Rho-YFP) was used as a positive control. A 293A cell line stably expressing WT or P23H RHO was prepared, and functional analysis was performed in this cell line. Normal rhodopsin proteins migrate to and localizes at the cell membrane. However, rhodopsin with P23H mutation does not fold properly and does not localize at the cell membrane but stays in the endoplasmic reticulum. Therefore, by identifying the location of the protein, the functional change of the protein can be confirmed.

The RHO targeting ribozyme expression vector was transfected into stable cells. To confirm the trans-splicing effect, RNA was extracted, and RT-PCR was carried out.

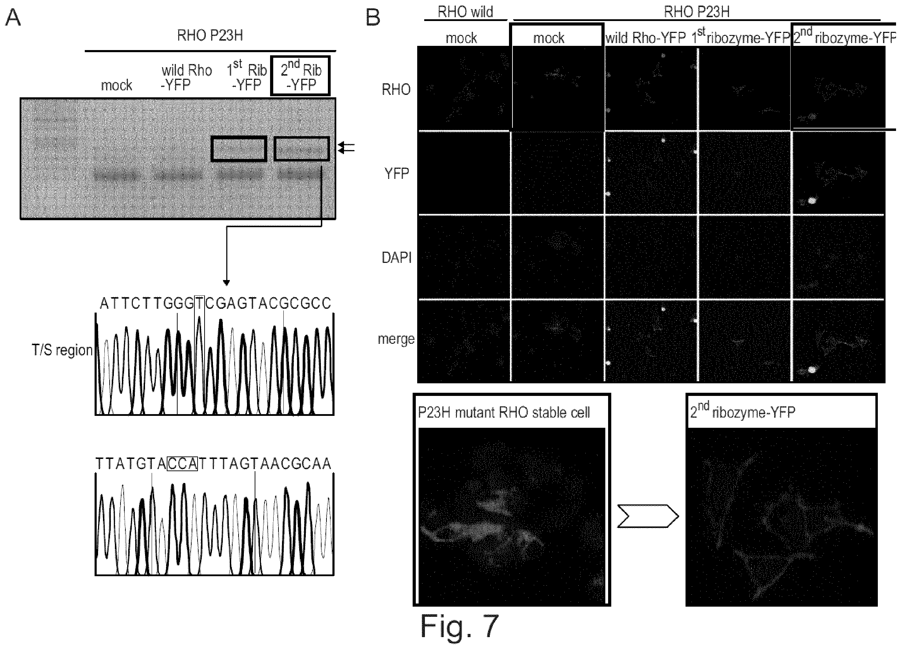

Specifically, 1×10⁵ stable cells were seeded in a 35 mm culture dish and grown in an incubator at 37°C with 5% CO₂. 2 µg of RHO-targeting ribozyme expression vector and 100 µl of Opti-MEM were mixed in a 1.5 ml tube. 2 µl of LIPOFECTAMINE^{®} 2000 and 100 µl of OPTI-MEM^{™} were mixed in another 1.5 ml tube and incubated at room temperature for 5 minutes. After that, the contents of the tubes were combined and mixed and incubated at room temperature for 20 minutes to allow the formation of a liposome complex. After 20 min, the tube was centrifuged for 10 sec. The mixture was added to plated cells for transfection and incubated for 4 hours, after which the transfection medium was replaced with fresh medium . The cells were then placed in an incubator for 48 hours at 37°C with 5% CO₂. After 48 hours, the cells were washed with 1X PBS and RNA was extracted with 500 µl of trizol. 5 µg of extracted RNA was treated with 1 µl of DNase I to remove gDNA. 1 µg of treated RNA was reverse transcribed to obtain cDNA. Using the synthesized cDNA, the trans-splicing product was amplified by PCR and purified. It was confirmed that the trans-splicing action occurred at the target site by nucleotide sequence analysis (Figure 7A).

In addition, after transfection of the RHO targeting ribozyme expression vector as described above, the cells were stained using an anti-RHO antibody and a secondary fluorescently labeled antibody. The intracellular distribution of RHO protein was analyzed by a fluorescence microscopy. It was confirmed that in the cells transfected with the RHO targeting ribozyme, RHO proteins were localized to the cell membrane, whereas the RHO proteins with P23H mutation were dispersed in cytosol (Figure 7B).

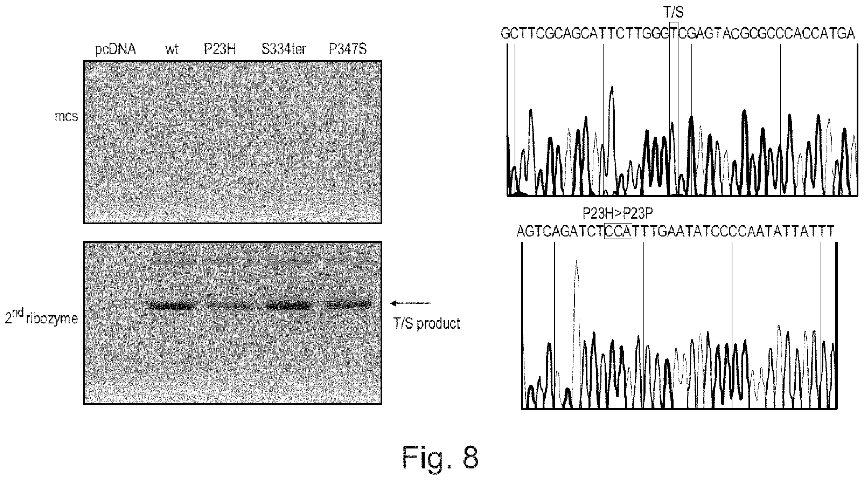

In addition, *in vitro* efficacy of RHO targeting ribozymes specific for various mutations was demonstrated in 293A cells by co-transfection (Figure 8). Specifically, 0.5 µg of the RHO-targeting ribozyme expression vector and 2 µg of expression vectors with various RHO mutant transcripts were mixed with 100 µl of Opti-MEM in a 1.5 ml tube. 2.5 µl of Lipofectamin2000 and 100 µl of Opti-MEM were mixed in another 1.5 ml tube and incubated at room temperature for 5 minutes. Cell transfection and RT-PCR were performed as described above. It was confirmed that mutant RHO transcripts were successfully replaced with normal RHO RNA by RHO-targeting ribozymes. Therefore, a trans-splicing ribozyme according to embodiments of the present invention can correct a specific RHO mutation by replacing the mutation site with WT sequence regardless of the type of mutation.

### Example 6. Recombinant Virus Construction and Confirmation of Trans-Splice Efficacy in Animal Models

### 6-1: Recombinant Virus Construction

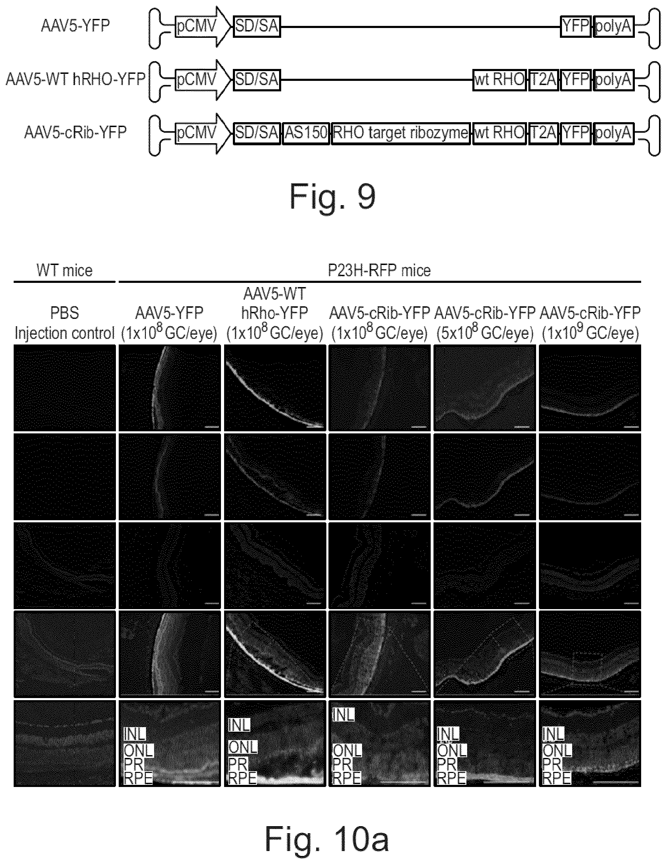

An expression vector was constructed using an Adeno-associated viruses (AAV) vector as a backbone, a RHO-targeting ribozyme of the second design of Example 4, and a CMV promoter (Figure 9). 20 µg of the backbone pAAV plasmid was digested with EcoRI and eluted on an agarose gel. After reacting DNA construct containing ribozyme with WT RHO synthesized using an infusion enzyme, transformed colonies were obtained. Each colony was cultured, plasmid was isolated and sequenced, and then clones with the correct cloned sequence were selected. The isolated plasmid was digested with EcoRV and Xbal, separated on the agarose gel and eluted. Synthesized YFP DNA was inserted using an infusion enzyme. Bacteria were transformed with the resulting construct. Plasmids were extracted from resulting colonies and correct cloning was confirmed by sequencing. In order to add the antisense sequence, selected constructs were digested with EcoRI, separated on an agarose gel and eluted. The AS 150 sequence obtained by PCR is reacted using an infusion enzyme to obtain colonies. After sequencing the colonies in the same way, the final AAV-cRib-YFP plasmid was obtained.

HEK-293T cells were triple transfected with the Helper vector, RHO ribozyme expression vector and Rep2Cap5 vector to produce an AAV viral vector. The transfected cells were lysed 72 hours post-transfection and supernatant was harvested using PEG precipitation. AAV viral vector was purified by density gradient ultracentrifugation using iodixanol.

### 6-2: Confirmation of the efficacy of trans-splicing ribozymes in animal models

The efficacy of ribozyme was confirmed in hP23H-RFP mouse model using the AAV expression vector of Example 6-1. hP23H mouse-RFP mouse model is a knocked-in model carrying an RFP-tagged human P23H RHO. It is a model widely used in RHO P23H studies. 1 µl of AAV expression vector was injected subretinally into eye of each mouse eye. The injection was performed using the IO kit with 34G needle and 1 µl of the vector was injected per eye into both eyes by scleral puncture. Upon completion of administration, antibiotics were injected into the eyes of the mouse and eye drops were used to prevent infection. The success or failure of administration was determined by confirming the formation of a bleb by FP/OCT imaging.

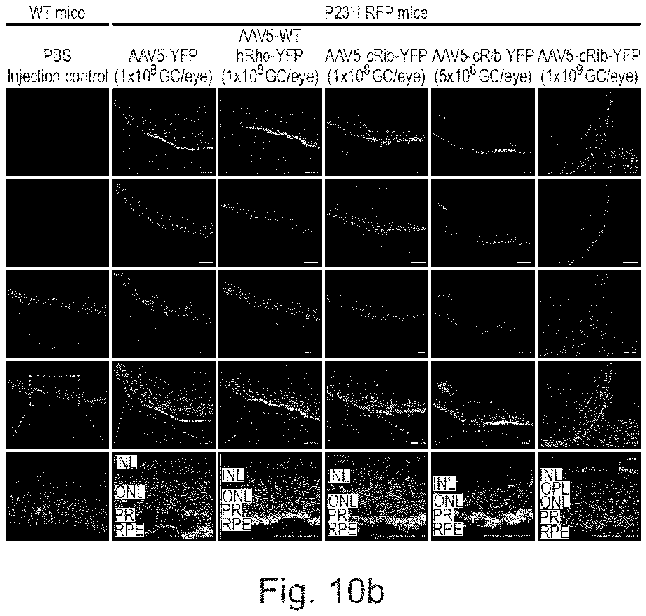

At 2 or 5 weeks after administration, the eyeballs were removed and tissue section slides were prepared. Cell nuclei were stained by DAPI. Staining of RHO protein itself was not performed because RHO was labeled with YFP. Figure 10 shows the results of 2 weeks after administration (Figure 10a) and 5 weeks after administration (Figure 10B).

AAV5-YFP (fluorescence positive) and AAV-WT hRho-YFP (gene, fluorescence positive) were used as a control for AAV5-cRib-YFP. Fluorescence of AAV5-YFP and AAV-WT hRho-YFP were observed also in retinal pigment epithelium (RPE), indicating that these were not introduced in a photoreceptor specific manner. On the other hand, in the case of AAV5-cRib-YFP carrying the RHO targeting ribozyme, no expression was observed in RPE and its expression was observed in photoreceptors only.

### Example 7: Assessment of Immune reaction and Inflammatory reaction after administration of trans-splicing ribozyme in animal model

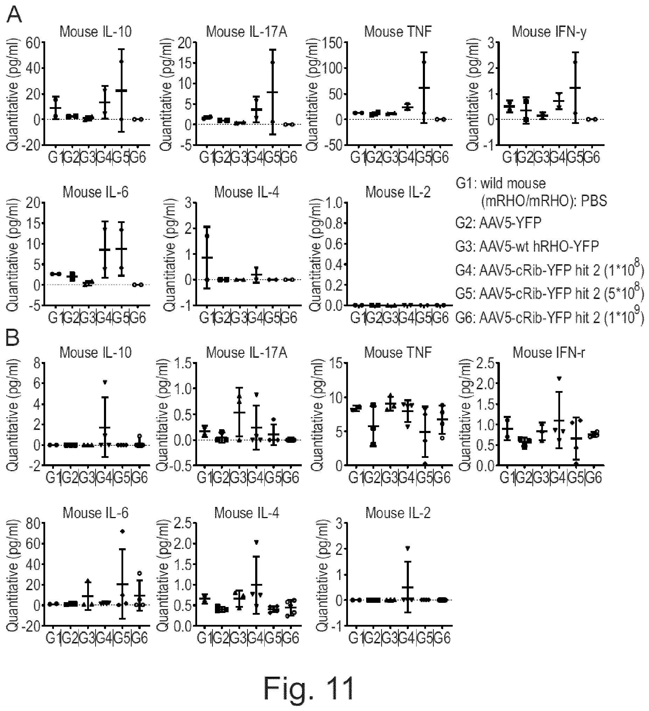

The results of assessing the immune and inflammatory responses in the serum of hP23H-RFP mice after administration of a trans-splicing ribozyme are shown in Figure 11 (Figure 11A: 2 weeks after administration; Figure11B: 5 weeks after administration).

An increase in IL-6, IL-17A, TNF-α, INF-γ, and IL-10 levels was observed at 2 weeks after AAV5-cRibYFP administration, and no changes in IL-4 and IL-2 levels were observed. Such changes were not observed in the AAV5-YFP and AAV-WT hRho-YFP-treated control groups, indicating that these changes are not attributed to viral vectors. Cytokines, which were increased at 2 weeks, returned (decreased) to levels of the control groups at the 5 weeks, indicating that inflammation and immune response are increased at the early subretinal administration and then returned to the original level at 5 weeks.

### Example 8: Confirmation of activity of ribozyme in retina and retinal pigment epithelium (RPE) tissue in an animal model after administration of trans-splicing ribozyme

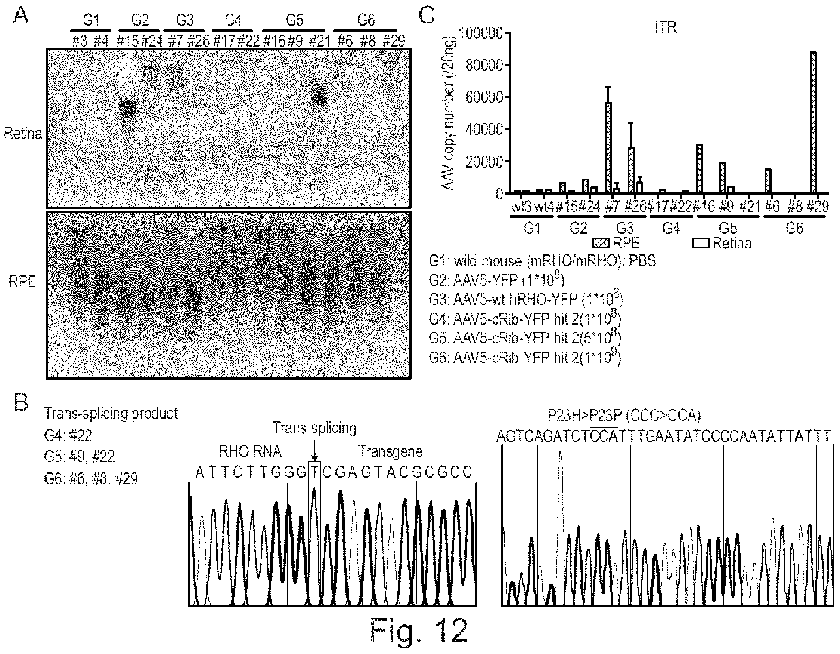

Occurrence of trans-splicing reaction through targeting RHO RNA in the photoreceptor was confirmed in the animal model after administration of a trans-splicing ribozyme. For this, the mouse eyeball was removed, retina and retinal pigment epithelial cells (RPE/choroid) were isolated, RNA was extracted and RT-PCR was performed to observe and confirm the production of trans-splicing products. The results are shown in Figure 12.

The amplified trans-splicing product PCR band could not be identified in a sample extracted from RPE. Retina-specific trans-splicing products were identified (Figure 12A). Sequencing was performed on the amplified trans-splicing product to confirm that the mutant RHO RNA was targeted and substituted with normal RHO RNA (Figure 12B).

In addition, when the AAV ITR region was amplified after extracting gDNA from the retina and retinal pigment epithelial (RPE) cells, higher levels were detected in retinal pigment epithelial cells than in the retina (Figure 12C), which indicates that AAV vectors are well delivered to RPE. However, transgene expression controlled by ribozyme was photoreceptor-specific.

### Example 9: Distribution of ribozymes in vivo after administration of trans-splicing ribozyme in mouse disease model

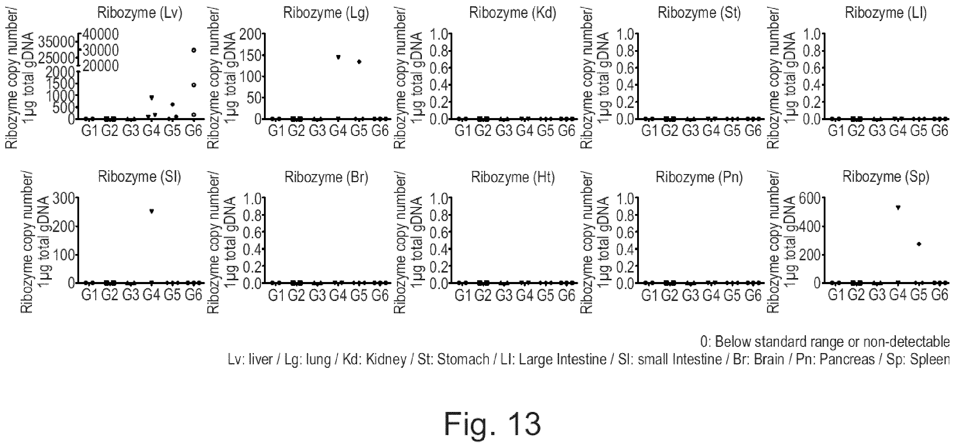

2 weeks after administration of ribozyme to hP23H-RFP mice, the distribution of ribozymes in organs was confirmed (Figure 13). The mice were sacrificed at 2 weeks after administration, whole organs were obtained, and gDNA was isolated and purified. Real-time PCR was performed using 1 µg of gDNA from each organ with a ribozyme-specific primer. Ribozymes were detected within a detection range in the liver of all animals administered with the trans-splicing ribozyme expression vector. In some animals, ribozymes were detected within a detection range in the lung, small intestine, and prostate. Ribozymes were not detected in other organs and tissues.

### Example 10: Toxicity analysis after administration of trans-splicing ribozyme to normal mice

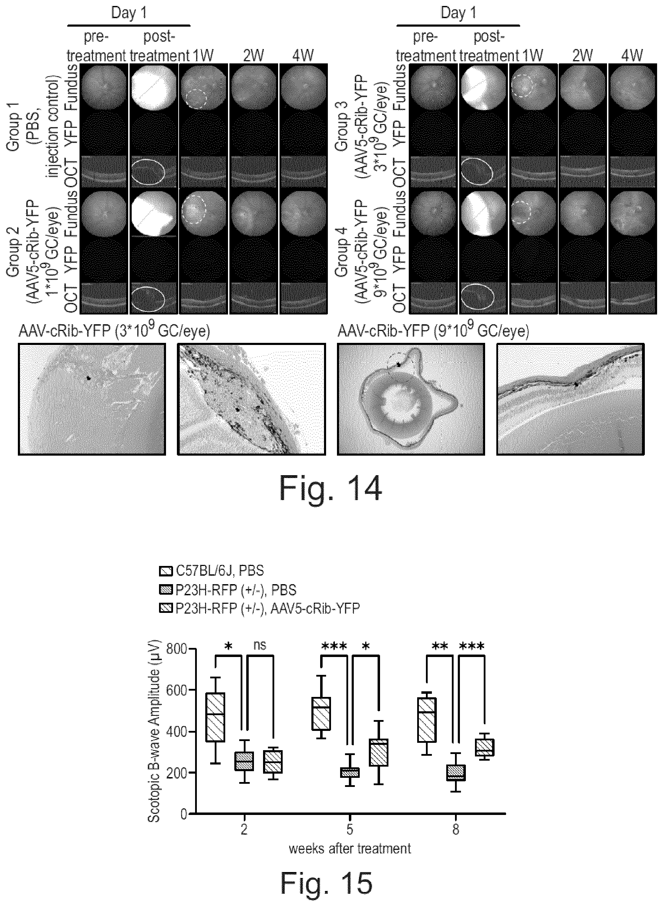

Toxicity after administration of trans-splicing ribozymes was assessed in normal mice (C57BL/6J). Images of fundus and OCT images were taken each week for 4 weeks after administration, and H&E staining was performed (Figure 14). It was confirmed that the bleb formed in the test group at the site of subretinal administration (red dotted line in Figure 14). Corneal inflammation and vitreous bleeding occurred in some individuals (white line). Lens degeneration, retinal degeneration and outer retina were observed in the groups treated with 3×10⁹ GC/eye by H&E staining. The occurrence of atrophy and histological inflammation of the choroid was also observed. No toxicity was observed at the concentration of less than 1×10⁹ GC/eye.

### Example 11: Electroretinogram after administration of trans-splicing ribozyme in mouse model

Electroretinogram (ERG) was performed at indicated time intervals after administration of trans-splicing ribozyme in 5 weeks-old hP23H-RFP mice (Figure 15 and Table 1). Scotopic-ERG responses were evaluated and amplitude of B-wave were compared. Mice were anesthetized with ketamine for electroretinogram evaluation. After additional local anesthesia, pupils of eye were dilated with eye drops. Mice were placed on the stage and electrodes were placed on the tail end, glabella, and retina, and ERG responses were recorded simultaneously from both eyes. Full-filed ERG were recorded at a flash intensity of 0.9 log cds/m² (10 responses/intensity). After the measurement was complete, one drop of antibiotic ophthalmic solution was administered into the mouse eye. 'LabScribeERG (iWorx DataAcquisition Software)' program was used for the analysis.

The P23H mutation of the rhodopsin leads to loss of normal function and visional impairment by inducing the death of rod cells and cone cells. It impairs the ability of retire photoreceptor cells (rods and cones) to convert light energy into electrical signals. As the number and function of photoreceptor cells decreases, the electrical signals diminish. The evaluation of these electrical signals was performed through electroretinography (ERG) to assess the difference in visual function. In electroretinography, B-wave is an indicator that determines whether an electrical signal is transmitted. B-wave was significantly increased at 5 weeks after administration of RHO-targeting ribozyme (AAV5-cRib-YFP) and this effect persisted until 8 weeks. Thus, maintenance and improvement of visual function was observed in disease models treated with AAV5-cRib-YFP.

**[Table 1] [0189]**

| **Scotopic B-wave Amplitude(µV)** | **C57BL/6J, PBS treatment** | **P23H-RFP (+/-), PBS treatment** | **P23H-RFP (+/-), AAV5-cRib-YFP treatment** |
|---|---|---|---|
| Week 2 after administration | **471.6 ± 139.12** | **258.2 ± 62.68** | **251.1 ± 56.08** |
| Week 5 after administration | **500.5 ± 101.22** | **204.7 ± 39.73** | **308.1 ± 92.87** |
| Week 8 after administration | **462.7 ± 116.38** | **193.6 ± 55.66** | **318.6 ± 46.17** |

### Example 12: Analysis of ribozyme activity and serum after administration of trans-splicing ribozyme in mouse model

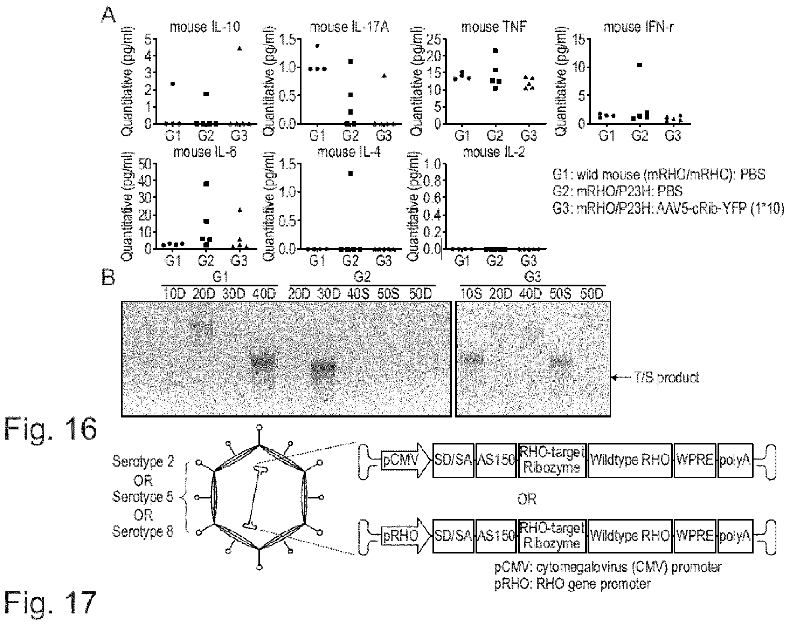

Serum analysis and ribozyme activity analysis were performed at 8 weeks after administration of trans-splicing ribozyme in hP23H-RFP mice (5 weeks old) (Figure 16). At week 8, when comparing normal animals (G1) administered with PBS and the animal model (G2) group administered only with PBS, no change in cytokines was observed by administration of trans-splicing ribozyme (Figure 16A). In Example 7, cytokine changes were observed at week 2 and then returned to a same level as normal control group animals at week 5. Thus, the results in this Example and results of Example 7 mean that the administration of the trans-splicing ribozyme does not affect cytokine changes even at week 8.

To assess whether or not RHO RNA-targeting trans-splicing action is maintained at week 8, RNA was extracted from the retina of the mice and presence of trans-splicing products was confirmed by RT-PCR (Figure 16B). The expression and action of AAV5-cRib-YFP was still observed at week 8. Thus, successful delivery of the recombinant AAV vector and maintenance of the ribozyme expression and activity in the retina was confirmed even at week 8.

### Example 13: Recombinant viral vector optimization

The ability of the various recombinant AAV vectors to infect and express in the retina was assessed (Figure 17). As shown in Figure 17, splicing donor/acceptor (SD/SA) sequence is linked to ribozyme at the 5' end and WPRE (Woodchuck hepatitis virus Posttranscriptional Regulatory Element) is linked at the 3' end. SD/SA and WPRE sequences are represented by SEQ ID NO: 11 and SEQ ID NO: 13, respectively. Various recombinant AAV serotypes and different promoters were used to construct different recombinant viral vectors. For example, as promoters, CMV promoter and the RHO promoter were used. And, to evaluate efficacy of the final candidate, YFP sequence was removed, and to increase expression, WPRE was inserted.

### 13-1: Assessment of ribozyme distribution and trans-splicing efficiency

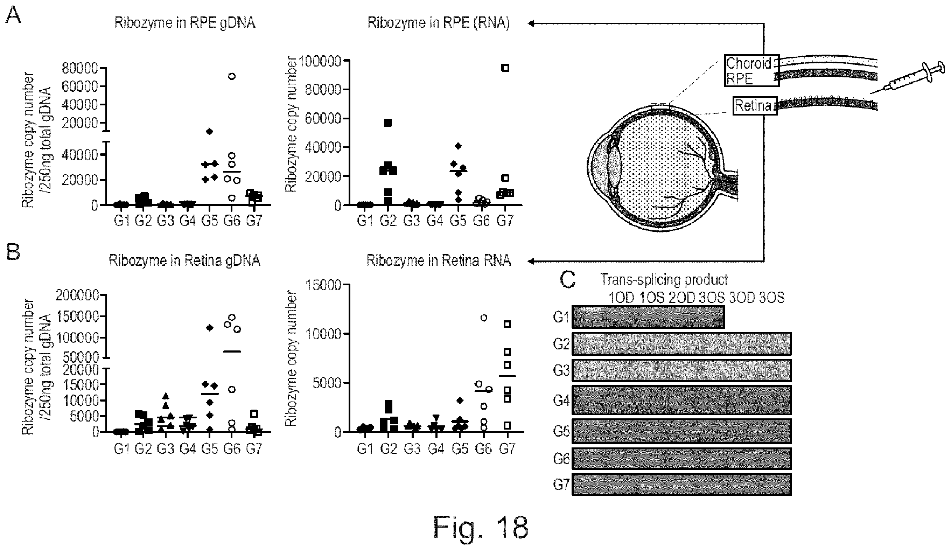

3 weeks after administration of the prepared recombinant AAV vector in 5 weeks old hP23H-RFP mice, ribozyme distribution and trans-splicing were evaluated (Figure 18).

The configurations of G1 to G7 are shown in Figure 18 and in Table 2 below.

**[Table 2] [0203]**

| Group | Animal | Tested Substance | |
|---|---|---|---|
| G1 | | PBS | |
| G2 | P23H-RFP(+/-) | AAV2/5-cRib-YFP | AAV serotype 5, CMV Promoter |
| G3 | | AAV2/2 cRib-WPRE | AAV serotype 2, CMV Promoter |
| G4 | | AAV2/2 RL-Rib-WPRE | AAV serotype 2, RHO Promoter |
| G5 | | AAV2/5 cRib-WPRE | AAV serotype 5, CMV Promoter |
| G6 | | AAV2/5 RL-Rib-WPRE | AAV serotype 5, RHO Promoter |
| G7 | | AAV2/8 cRib-WPRE | AAV serotype 8, CMV Promoter |

In order to check the infection rate for each serotype, the eyeballs of treated mice were removed 3 weeks after injection. Retina and RPE/choroid were separated and RNA and gDNA were extracted. When the extracted gDNA was compared, AAV serotype 5 showed more efficient transduction to both the retina and the RPE, than serotype 2 or 8. Parallel RNA analysis was performed by RT-PCR. The trans-splicing ribozyme was highly expressed in RPE as well under the CMV promoter regulation, while, in the retina, the ribozyme was highly expressed under the RHO promoter regulation compared to the ribozyme expression under the CMV promoter regulation (Figures 18A and 18B).

The presence of trans-splicing products through the trans-splicing action of the ribozyme in the retina was detected and confirmed by PCR. Then, the PCR bands of trans-splicing products formed by AAV serotype 5 under the RHO promoter regulation were dense (Figure 18C).

The results indicate that AAV serotype 5 RL-Rib-WPRE showed excellent delivery and expression in the retina as well as excellent trans-splicing efficiency.

### 13-2: Electroretinogram after administration of trans-splicing ribozyme in mouse model

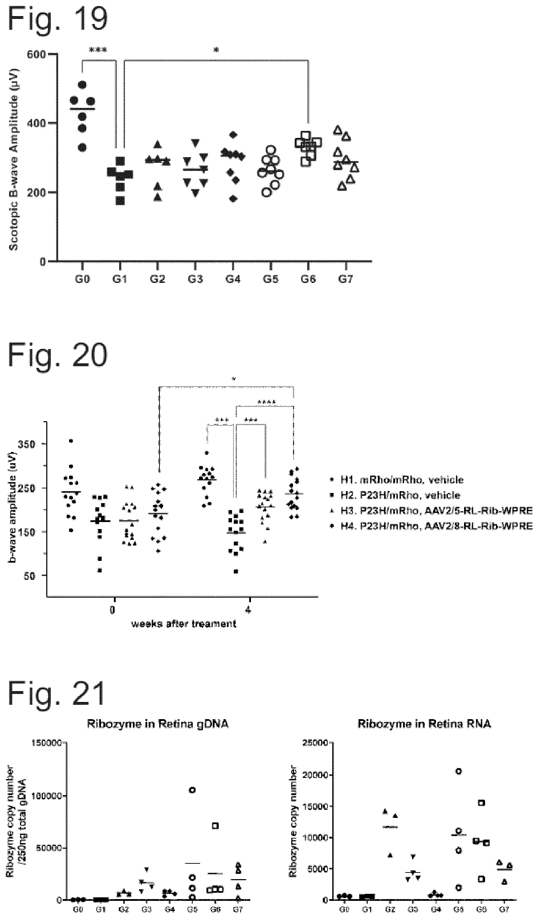

Electroretinogram examination was conducted at 6 weeks after administration of trans-splicing ribozyme in hP23H-RFP mice (5 weeks of age). The results are shown in Figure 19 and Table 3.

**[Table 3]**

| Group | Animal | Tested Substance | B-wave(µV) |
|---|---|---|---|
| G0 | C57BL/6J (mRHO/mRHO) | PBS | 428.7±65.15 |
| G1 | P23H-RFP(+/-) | PBS | 240.1±39.67 |
| G2 | | AAV2/5-cRib-YFP | 272.1±56.75 |
| G3 | | AAV2/2 cRib-WPRE | 263.7±40.46 |
| G4 | | AAV2/2 RL-Rib-WPRE | 284.9±64.47 |
| G5 | | AAV2/5 cRib-WPRE | 263.4±37.69 |
| G6 | | AAV2/5 RL-Rib-WPRE | 318.1±26.35 |
| G7 | | AAV2/8- cRib-WPRE | 296.2±57.86 |

B-wave amplitude of P23H-RFP mice (G1) was significantly decreased compared to the normal wild-type mice (G0). This result is consistent with the photoreceptor cells damage in P23H-RFP mice caused by the RHO mutation resulting impaired signal transmission.

When the electroretinogram was taken after administration of each AAV to the hP23H-RFP mice, it was confirmed that the average scotopic B-wave amplitude in all the test groups increased compared to the PBS-treated group (G1). Comparison between promoters showed that B-wave amplitude was significantly higher when RHO promoter was used than when CMV promoter was used (G3<G4, G5<G6), compared to the control group administered with PBS (G1). Comparison between serotypes, both AAV2/5 serotypes (G5, G6) and the AAV2/8 group (G7) showed significantly increased B-wave amplitude, compared to the PBS administration group (G1). The AAV2/5 RL-Rib-WPRE administration group exhibited the most significantly increased B-wave, indicating that subretinal administration of AAV2/5 RL-Rib-WPRE could show the highest effect of improving the visual function of the disease model.

Electroretinogram was performed at 4 weeks after administration of the following recombinant AAV vectors to 5 weeks-old hP23H-RFP mice: AAV2/5 RL-Rib-WPRE and AAV2/8 RL-Rib-WPRE, recombinant AAV vectors of serotype 5 and 8 with RHO promoter, respectively. The results in Figure 20 and Table 4.

**[Table 4] [0218]**

| Group | Animal | Tested Substance | B-wave(µV) (0 week) | B-wave(µV) (4 week) |
|---|---|---|---|---|
| H1 | C57BL/6J (mRHO/mRHO) | PBS | 240.3±52.45 | 267.8±33.37 |
| H2 | P23H-RFP(+/-) | PBS | 173.7±50.60 | 146.8±41.18 |
| H3 | | AAV2/5 RL-Rib-WPRE | 176.1±44.53 | 206.2±33.59 |
| H4 | | AAV2/8 RL-Rib-WPRE | 191.5±47.24 | 235.8±36.06 |

As shown in Table 4 and Figure 20, the B-wave amplitude at 4 weeks after administration was significantly higher in AAV2/5 RL-Rib-WPRE (H3) group and AAV2/8 RL-Rib-WPRE group (H4), compared to PBS-treated group (H2). Considering that AAV2/8 RL-Rib-WPRE group (H4) showed higher B-wave before administration (0 week) than the PBS control group (H2) and the AAV2/5 RL-Rib-WPRE administration group (H3), we speculate that B-wave increasing ability of the AAV2/5 RL-Rib-WPRE administration group (H3) and the AAV2/8 RL-Rib-WPRE administration group (H4) were similar. Therefore, administration of AAV2/5 RL-Rib-WPRE and AAV2/8 RL-Rib-WPRE significantly improved visual function in this mouse model compared to the PBS-administered control group (H2).

### 13-3: Distribution and trans-splicing activity of ribozyme in retinal after trans-splicing ribozyme administration in mouse model

The distribution of ribozyme in the retina and trans-splicing activity was assessed 6 weeks after administration of ribozyme to 5 week old hP23H-RFP mice as shown in Figure 21 and Table 5.

**[Table 5] [0225]**

| Group | Animal | Tested Substance |
|---|---|---|
| G0 | C57BL/6J (mRHO/mRHO) | PBS |
| G1 | P23H-RFP(+/-) | PBS |
| G2 | | AAV2/5-cRib-YFP |
| G3 | | AAV2/2 cRib-WPRE |
| G4 | | AAV2/2 RL-Rib-WPRE |
| G5 | | AAV2/5 cRib-WPRE |
| G6 | | AAV2/5 RL-Rib-WPRE |
| G7 | | AAV2/8- cRib-WPRE |

After confirming the functional efficacy in mouse model, the retinal gDNA and RNA was isolated and analyzed. AAV serotype 5 showed the same results as those seen at week 3 in Example 13-1 and it was confirmed that infection of the retina was successful. RNA analysis showed good ribozyme expression with AAV serotype 5 with RHO promoter. Delivery and expression of AAV2/5 RL-Rib-WPRE in the retina was stable at week 6.

Summarizing the above results, it was confirmed that AAV serotypes 5 and 8 was efficiently delivered into both retinal and RPE and the trans-splicing ribozyme regulated by RHO promoter was specifically expressed in the retina in the hP23H disease model.

Expression of trans-splicing ribozyme according to the present invention was maintained from week 3 of the initial administration to 6 week after administration, and improvement of visual function was observed as measured by electroretinography at weeks 4 and 6 after administration.

Aspects of the present invention has been described in detail above. Those of ordinary skill in the art would understand that these specific techniques are merely some of the preferred implementations. It is to be understood that the description above does not limit the scope of the present invention. The substantial scope of the present invention is defined by the claims and includes the equivalents.

## Claims

1. A trans-splicing ribozyme targeting a rhodopsin transcript.

2. The trans-splicing ribozyme according to claim 1, wherein the trans-splicing ribozyme has a structure of 5'-IGS (internal guide sequence)-Ribozyme^{∗}-3'.

3. The trans-splicing ribozyme according to claim 2 further comprising an exon sequence at a position 3'- direction of the Ribozyme^{∗}.

4. The trans-splicing ribozyme according to claim 2, wherein the IGS comprises 5-10 consecutive nucleotides in length and is capable of complementarily binding a rhodopsin transcript.

5. The trans-splicing ribozyme according to claim 4, wherein the IGS comprises a consecutive nucleotide sequence of 5-10 nt in length and is capable of complementarily binding at the rhodopsin transcript regions comprising nucleotides at the following positions: +30, +35, +42, +43, +52, +54, +55, +59, +75, +97, +116, +122, +123, +127, +132, +140, +154, +165, +171, +187, +191, +207, +215, +222, +230, +232, +244, +256, +262, +273, +298, +308, +381, +403, +661 or +688.

6. The trans-splicing ribozyme according to claim 1, wherein the rhodopsin transcript comprises a mutation.

7. The trans-splicing ribozyme according to claim 6, wherein the mutation in the rhodopsin transcript is mutation at one or more positions ranging from position 1 to position 1142 of SEQ ID NO: 1.

8. The trans-splicing ribozyme according to claim 7, wherein the mutation is one or more selected from the group consisting of: L328P, T342M, Q344R/P/ter, V345L/M, A346P, P347A/R/Q/L/S/T, ter349/Q/E, N15S, T17M, V20G, P23A/H/L, Q28H, G51R/V, P53R, T58R/M, V87D/L, G89D, G106R/W, C110F/R/S/Y, E113K, L125R, W161R, A164E/V, C167R/W, P171Q/L/S, Y178N/D/C, E181K, G182S/V, C185R, C187G/Y, G188R/E, D190N/G/Y, H211R/P, C222R, P267R/L, S270R, K296N/E/M, R135G/L/P/W, T4K, T17M, M39R, N55K, G90V, M44T, V137M, G90D, T94I, A292E, A295V, F45L, V209M, F220C, P12R, R21C, Q28H, L40R, L46R, L47R, F52Y, F56Y, L57R, Y60ter, Q64ter, R69H, N78I, L79P, L88P, T92I, T97I, V104F, G109R, G114D/V, E122G, W126L/ter, S127F, L131P, Y136ter, C140S, T160T, M163T, A169P, P170H/R, S176F, P180A/S, Q184P, S186P/W, Y191C, T193M, M207R/K, V210F, I214N, P215L/T, M216R/L/K, R252P, T289P, S297R, A298D, K311E, N315ter, E341K, S343C, and Q312ter.

9. The trans-splicing ribozyme according to claim 1, wherein the trans-splicing ribozyme comprises the sequence of SEQ ID NOs: 2 to 4.

10. A non-viral gene carrier comprising the trans-splicing ribozyme of claim 1.

11. A gene construct comprising the trans-splicing ribozyme of claim 1.

12. The gene construct of claim 11 further comprising a desired gene 3'-end direction of the trans-splicing ribozyme.

13. The gene construct of claim 12, wherein the desired gene is a normal rhodopsin gene or a reporter gene.

14. The gene construct of claim 11 further comprising an antisense sequence at a position 5'-end direction of the trans-splicing ribozyme, said antisense sequence being complimentary to a portion of the target rhodopsin transcript sequence.

15. The gene construct according to any one of claims 11 - 14, further comprising a promoter sequence operably linked to the trans-splicing ribozyme.

16. A recombinant expression vector comprising the gene construct according to any one of claims 11 - 14.

17. A recombinant virus comprising the gene construct according to any one of claims 11-14.

18. A non-viral gene carrier comprising the gene construct according to any one of claims 11-14.

19. The recombinant virus of claim 17, wherein the virus is selected from the group consisting of adenovirus, adeno-associated virus (AAV), retrovirus, lentivirus, herpes simplex virus, and vaccinia virus.

20. The recombinant virus of claim 17, wherein the virus is a recombinant adeno-associated virus (AAV) comprising a polynucleotide encoding the trans-splicing ribozyme of claim 1, wherein the polynucleotide is operably linked to a promoter sequence; and wherein the recombinant AAV is derived from a native or artificial adenovirus serotype, an isolate thereof, or a clade thereof.

21. A pharmaceutical composition for preventing or treating retinitis pigmentosa, comprising the trans-splicing ribozyme according to claim 1, the non-viral gene carrier according to claim 10, the gene construct according to any one of claims 11-14, the recombinant expression vector according to claim 16, the recombinant virus according to claim 17, or the non-viral gene carrier according to claim 18, as an active ingredient.

22. A method of treatment or prevention of retinitis pigmentosa comprising administering the pharmaceutical composition of claim 21 to a subject in need thereof.


<110>    Rznomics Inc.

<120>    A trans-splicing ribozyme specific for rhodopsin transcript and
         uses thereof

<130>    DHP20-471

<150>    KR 20/092,265
<151>    2020-07-24

<160>    13

<170>    KoPatentIn 3.0

<210>    1
<211>    2768
<212>    DNA
<213>    Homo sapiens

<220>
<221>    gene
<222>    (1)..(2768)
<223>    Homo sapiens rhodopsin (RHO)

<400>    1
agagtcatcc agctggagcc ctgagtggct gagctcaggc cttcgcagca ttcttgggtg        60

ggagcagcca cgggtcagcc acaagggcca cagccatgaa tggcacagaa ggccctaact       120

tctacgtgcc cttctccaat gcgacgggtg tggtacgcag ccccttcgag tacccacagt       180

actacctggc tgagccatgg cagttctcca tgctggccgc ctacatgttt ctgctgatcg       240

tgctgggctt ccccatcaac ttcctcacgc tctacgtcac cgtccagcac aagaagctgc       300

gcacgcctct caactacatc ctgctcaacc tagccgtggc tgacctcttc atggtcctag       360

gtggcttcac cagcaccctc tacacctctc tgcatggata cttcgtcttc gggcccacag       420

gatgcaattt ggagggcttc tttgccaccc tgggcggtga aattgccctg tggtccttgg       480

tggtcctggc catcgagcgg tacgtggtgg tgtgtaagcc catgagcaac ttccgcttcg       540

gggagaacca tgccatcatg ggcgttgcct tcacctgggt catggcgctg gcctgcgccg       600

cacccccact cgccggctgg tccaggtaca tccccgaggg cctgcagtgc tcgtgtggaa       660

tcgactacta cacgctcaag ccggaggtca acaacgagtc ttttgtcatc tacatgttcg       720

tggtccactt caccatcccc atgattatca tctttttctg ctatgggcag ctcgtcttca       780

ccgtcaagga ggccgctgcc cagcagcagg agtcagccac cacacagaag gcagagaagg       840

aggtcacccg catggtcatc atcatggtca tcgctttcct gatctgctgg gtgccctacg       900

ccagcgtggc attctacatc ttcacccacc agggctccaa cttcggtccc atcttcatga       960

ccatcccagc gttctttgcc aagagcgccg ccatctacaa ccctgtcatc tatatcatga      1020

tgaacaagca gttccggaac tgcatgctca ccaccatctg ctgcggcaag aacccactgg      1080

gtgacgatga ggcctctgct accgtgtcca agacggagac gagccaggtg gccccggcct      1140

aagacctgcc taggactctg tggccgacta taggcgtctc ccatccccta caccttcccc 1200

cagccacagc catcccacca ggagcagcgc ctgtgcagaa tgaacgaagt cacataggct 1260

ccttaatttt tttttttttt ttaagaaata attaatgagg ctcctcactc acctgggaca 1320

gcctgagaag ggacatccac caagacctac tgatctggag tcccacgttc cccaaggcca 1380

gcgggatgtg tgcccctcct cctcccaact catctttcag gaacacgagg attcttgctt 1440

tctggaaaag tgtcccagct tagggataag tgtctagcac agaatggggc acacagtagg 1500

tgcttaataa atgctggatg gatgcaggaa ggaatggagg aatgaatggg aagggagaac 1560

atatctatcc tctcagaccc tcgcagcagc agcaactcat acttggctaa tgatatggag 1620

cagttgtttt tccctccctg ggcctcactt tcttctccta taaaatggaa atcccagatc 1680

cctggtcctg ccgacacgca gctactgaga agaccaaaag aggtgtgtgt gtgtctatgt 1740

gtgtgtttca gcactttgta aatagcaaga agctgtacag attctagtta atgttgtgaa 1800

taacatcaat taatgtaact agttaattac tatgattatc acctcctgat agtgaacatt 1860

ttgagattgg gcattcagat gatggggttt cacccaacct tggggcaggt ttttaaaaat 1920

tagctaggca tcaaggccag accagggctg ggggttgggc tgtaggcagg gacagtcaca 1980

ggaatgcaga atgcagtcat cagacctgaa aaaacaacac tgggggaggg ggacggtgaa 2040

ggccaagttc ccaatgaggg tgagattggg cctggggtct cacccctagt gtggggcccc 2100

aggtcccgtg cctccccttc ccaatgtggc ctatggagag acaggccttt ctctcagcct 2160

ctggaagcca cctgctcttt tgctctagca cctgggtccc agcatctaga gcatggagcc 2220

tctagaagcc atgctcaccc gcccacattt aattaacagc tgagtccctg atgtcatcct 2280

tatctcgaag agcttagaaa caaagagtgg gaaattccac tgggcctacc ttccttgggg 2340

atgttcatgg gccccagttt ccagtttccc ttgccagaca agcccatctt cagcagttgc 2400

tagtccattc tccattctgg agaatctgct ccaaaaagct ggccacatct ctgaggtgtc 2460

agaattaagc tgcctcagta actgctcccc cttctccata taagcaaagc cagaagctct 2520

agctttaccc agctctgcct ggagactaag gcaaattggg ccattaaaag ctcagctcct 2580

atgttggtat taacggtggt gggttttgtt gctttcacac tctatccaca ggatagattg 2640

aaactgccag cttccacctg atccctgacc ctgggatggc tggattgagc aatgagcaga 2700

gccaagcagc acagagtccc ctggggctag aggtggagga ggcagtcctg ggaatgggaa 2760

aaacccca 2768

<210> 2
<211> 405
<212> DNA
<213> Artificial Sequence

<220>
<223>    trans-splicing ribozyme


<400>    2
gctcccgccc aaaaaagtta tcaggcatgc acctggtagc tagtctttaa accaatagat      60

tgcatcggtt taaaaggcaa gaccgtcaaa ttgcgggaaa ggggtcaaca gccgttcagt     120

accaagtctc aggggaaact ttgagatggc cttgcaaagg gtatggtaat aagctgacgg     180

acatggtcct aaccacgcag ccaagtccta agtcaacaga tcttctgttg atatggatgc     240

agttcacaga ctaaatgtcg gtcggggaag atgtattctt ctcataagat atagtcggac     300

ctctccttaa tgggagctag cggatgaagt gatgcaacac tggagccgct gggaactaat     360

ttgtatgcga aagtatattg attagttttg gagtactcgg ggagc                     405


<210>    3
<211>    409
<212>    DNA
<213>    Artificial Sequence

<220>
<223>    trans-splicing ribozyme


<400>    3
cgtactccgc ccaaaaaagt tatcaggcat gcacctggta gctagtcttt aaaccaatag      60

attgcatcgg tttaaaaggc aagaccgtca aattgcggga aaggggtcaa cagccgttca     120

gtaccaagtc tcaggggaaa ctttgagatg gccttgcaaa gggtatggta ataagctgac     180

ggacatggtc ctaaccacgc agccaagtcc taagtcaaca gatcttctgt tgatatggat     240

gcagttcaca gactaaatgt cggtcgggga agatgtattc ttctcataag atatagtcgg     300

acctctcctt aatgggagct agcggatgaa gtgatgcaac actggagccg ctgggaacta     360

atttgtatgc gaaagtatat tgattagttt tggagtactc gcgagtacg                 409


<210>    4
<211>    409
<212>    DNA
<213>    Artificial Sequence

<220>
<223>    trans-splicing ribozyme


<400>    4
cgtactccgc ccaaaaaagt tatcaggcat gcacctggta gctagtcttt aaaccaatag      60

attgcatcgg tttaaaaggc aagaccgtca aattgcggga aaggggtcaa cagccgttca     120

gtaccaagtc tcaggggaaa ctttgagatg gccttgcaaa gggtatggta ataagctgac     180

ggacatggtc ctaaccacgc agccaagtcc taagtcaaca gatcttctgt tgatatggat     240

gcagttcaca gactaaatgt cggtcgggga agatgtattc ttctcataag atatagtcgg 300

acctctcctt aatgggagct agcggatgaa gtgatgcaac actggagccg ctgggaacta 360

atttgtatgc gaaagtatat tgattagttt tggagtactc gccagtacg 409

<210> 5
<211> 387
<212> DNA
<213> Artificial Sequence

<220>
<223> Ribozyme*

<400> 5
aaaagttatc aggcatgcac ctggtagcta gtctttaaac caatagattg catcggttta 60

aaaggcaaga ccgtcaaatt gcgggaaagg ggtcaacagc cgttcagtac caagtctcag 120

gggaaacttt gagatggcct tgcaaagggt atggtaataa gctgacggac atggtcctaa 180

ccacgcagcc aagtcctaag tcaacagatc ttctgttgat atggatgcag ttcacagact 240

aaatgtcggt cggggaagat gtattcttct cataagatat agtcggacct ctccttaatg 300

ggagctagcg gatgaagtga tgcaacactg gagccgctgg gaactaattt gtatgcgaaa 360

gtatattgat tagttttgga gtactcg 387

<210> 6
<211> 6
<212> RNA
<213> Artificial Sequence

<220>
<223> IGS

<400> 6
gcccaa 6

<210> 7
<211> 9
<212> RNA
<213> Artificial Sequence

<220>
<223> P1

<400> 7
cccgcccaa 9

<210> 8
<211> 7
<212> RNA
<213> Artificial Sequence

<220>
<223>    P10


<400>    8
cguacuc                                                                  7


<210>    9
<211>    7
<212>    RNA
<213>    Artificial Sequence

<220>
<223>    P10


<400>    9
gaguacg                                                                  7


<210>    10
<211>    150
<212>    DNA
<213>    Artificial Sequence

<220>
<223>    AS150


<400>    10
ggcggccagc atggagaact gccatggctc agccaggtag tactgtgggt actcgaagtg      60

gctgcgtacc acacccgtcg cattggagaa gggcacgtag aagttagggc cttctgtgcc     120

attcatggct gtggcccttg tggctgaccc                                      150


<210>    11
<211>    400
<212>    DNA
<213>    Artificial Sequence

<220>
<223>    SD/SA


<400>    11
aaaaaatgct ttcttctttt aatatacttt tttgtttatc ttatttctaa tactttccct      60

aatctctttc tttcagggca ataatgatac aatgtatcat gcctctttgc accattctaa     120

agaataacag tgataatttc tgggttaagg caatagcaat atttctgcat ataaatattt     180

ctgcatataa attgtaactg atgtaagagg tttcatattg ctaatagcag ctacaatcca     240

gctaccattc tgcttttatt ttatggttgg gataaggctg gattattctg agtccaagct     300

aggccctttt gctaatcatg ttcatacctc ttatcttcct cccacagctc ctgggcaacg     360

tgctggtctg tgtgctggcc catcactttg gcaaagaatt                           400

<210>    12
<211>    36
<212>    DNA
<213>    Artificial Sequence

<220>
<223>    5′ UTR


<400>    12
gggagcagcc acgggtcagc cacaagggcc acagcc                                36


<210>    13
<211>    589
<212>    DNA
<213>    Artificial Sequence

<220>
<223>    WPRE


<400>    13
aatcaacctc tggattacaa aatttgtgaa agattgactg gtattcttaa ctatgttgct        60

ccttttacgc tatgtggata cgctgcttta atgcctttgt atcatgctat tgcttcccgt       120

atggctttca ttttctcctc cttgtataaa tcctggttgc tgtctcttta tgaggagttg       180

tggcccgttg tcaggcaacg tggcgtggtg tgcactgtgt ttgctgacgc aacccccact       240

ggttggggca ttgccaccac ctgtcagctc ctttccggga ctttcgcttt ccccctccct       300

attgccacgg cggaactcat cgccgcctgc cttgcccgct gctggacagg ggctcggctg       360

ttgggcactg acaattccgt ggtgttgtcg gggaaatcat cgtcctttcc ttggctgctc       420

gcctgtgttg ccacctggat tctgcgcggg acgtccttct gctacgtccc ttcggccctc       480

aatccagcgg accttccttc ccgcggcctg ctgccggctc tgcggcctct tccgcgtctt       540

cgccttcgcc ctcagacgag tcggatctcc ctttgggccg cctccccgc                   589